(19) Europäisches Patentamt
European Patent Office
Office européen des brevets

(11) Numéro de publication : **0 423 011 B1**

(12) # FASCICULE DE BREVET EUROPEEN

(45) Date de publication du fascicule du brevet : **20.10.93 Bulletin 93/42**

(51) Int. Cl.$^5$ : **A61K 7/00**

(21) Numéro de dépôt : **90402788.5**

(22) Date de dépôt : **08.10.90**

(54) **Composition cosmétique ou pharmaceutique pour application topique contenant au moins un dérivé rétinoide et au moins un dérivé de pyrimidine.**

(30) Priorité : **12.10.89 FR 8913358**

(43) Date de publication de la demande : **17.04.91 Bulletin 91/16**

(45) Mention de la délivrance du brevet : **20.10.93 Bulletin 93/42**

(84) Etats contractants désignés : **AT BE CH DE DK ES FR GB GR IT LI NL SE**

(56) Documents cités :
**EP-A- 0 229 561**
**WO-A-88/07362**
**FR-A- 2 619 309**
**GB-A- 2 189 457**

(73) Titulaire : **L'OREAL**
**14, Rue Royale**
**F-75008 Paris (FR)**

(72) Inventeur : **Grollier, Jean-François**
**16 Bis, Boulevard Morland**
**F-75004 Paris (FR)**
Inventeur : **Rosenbaum, Georges**
**2, rue J. H. Mansart**
**F-92600 Asnieres (FR)**
Inventeur : **Hansenne-Richoux, Isabelle**
**19, rue Boursault**
**F-75017 Paris (FR)**

(74) Mandataire : **Peuscet, Jacques et al**
**Cabinet Peuscet 68, rue d'Hauteville**
**F-75010 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne une composition contenant, en association, au moins un dérivé rétinoïde et au moins un dérivé de pyrimidine,qui est stable à la lumière et au stockage. Cette composition peut être utilisée comme médicament ou comme cosmétique pour induire et stimuler la croissance des cheveux et pour diminuer leur chute.

L'homme a un capital de 100 000 à 150 000 cheveux et il est normal de perdre quotidiennement 50 à 100 cheveux. La maintenance de ce capital résulte essentiellement du fait que la vie d'un cheveu est soumise à un cycle pilaire au cours duquel le cheveu se forme, croît et tombe avant d'être remplacé par un nouveau cheveu qui apparaît dans le même follicule. On observe,au cours d'un cycle pilaire,successivement trois phases, à savoir : la phase anagène, la phase catagène et la phase télogène. Au cours de la première phase, dite anagène, le cheveu passe par une période de croissance active associée à une intense activité métabolique au niveau du bulbe. La deuxième phase dite catagène est transitoire et elle est marquée par un ralentissement des activités mitotiques. Au cours de cette phase, le cheveu subit une évolution, le follicule s'atrophie et son implantation dermique apparaît de plus en plus haute. La phase terminale dite télogène correspond à une période de repos du follicule et le cheveu finit par tomber poussé par un cheveu anagène naissant. Ce processus de renouvellement physique permanent subit une évolution naturelle au cours du vieillissement, les cheveux deviennent plus fins et leurs cycles plus courts. L'alopécie survient lorsque ce processus de renouvellement physique est accéléré ou perturbé, c'est-à-dire que les phases de croissance sont raccourcies, le passage des cheveux en phase télogène est plus précoce et les cheveux tombent en plus grand nombre. Les cycles de croissance successifs aboutissent à des cheveux de plus en plus fins et de plus en plus courts, se transformant peu à peu en un duvet non pigmenté qui peut conduire à la calvitie. Le cycle pilaire est tributaire par ailleurs de nombreux facteurs pouvant entraîner une alopécie plus ou moins prononcée. Parmi ces facteurs, on peut citer les facteurs alimentaires, endocriniens et nerveux.

On recherche depuis de nombreuses années dans l'industrie cosmétique ou pharmaceutique des compositions permettant de supprimer ou de réduire l'effet de l'alopécie et notamment d'induire ou de stimuler la croissance des cheveux et de diminuer leur chute. On cherche, notamment, à prolonger la phase anagène du cycle pilaire par rapport à la phase télogène qui, comme indiqué ci-dessus, conduit à la chute des cheveux. Il est connu que certains dérivés de pyrimidine, en particulier le dérivé généralement appelé "minoxidil", favorisent la croissance des cheveux.

Dans la demande WO-A-83-02558, on a déjà proposé d'utiliser des dérivés rétinoïdes, en particulier l'acide tout-trans rétinoïque ou trétinoïne, associés au minoxidil pour le traitement des cheveux. L'association de ces deux produits améliore, par rapport au minoxidil seul, la vitesse de croissance des cheveux, prolonge la phase anagène du cycle pilaire et permet de traiter certaines alopécies. Dans ces lotions pour application topique, le rétinoïde, qui n'est pas soluble dans l'eau, est généralement en solution dans l'alcool éthylique ou le propylèneglycol. Malheureusement, la présence de ces solvants donne à la lotion un caractère irritant vis-a-vis de la peau. En outre, les rétinoïdes, et notamment l'acide tout-trans rétinoïque, présentent une certaine instabilité à la lumière et l'on a constaté que la présence du minoxidil dans des compositions d'acide tout-trans-rétinoïque accélère la dégradation du rétinoïde à la lumière et/ou au cours du stockage.

On a proposé, pour remédier à ce dernier inconvénient, de stocker séparément le dérivé rétinoïque et le dérivé pyrimidique (FR-A-2 619 309) et de les appliquer séparément successivement. Mais ce traitement est relativement compliqué pour l'utilisateur car il nécessite l'emploi de deux conditionnements et des applications à intervalles déterminés.

La demanderesse a maintenant découvert une composition contenant, en association, au moins un dérivé rétinoïde et au moins un dérivé de pyrimidine, qui peut être stockée sans qu'il y ait dégradation du dérivé rétinoïde. De plus, la composition a l'avantage d'être en milieu aqueux et de ne pas contenir de solvants ayant un caractère irritant pour la peau.

La présente invention a pour objet une composition cosmétique ou pharmaceutique pour application topique contenant, en association, au moins un dérivé rétinoïde et au moins un dérivé de pyrimidine, caractérise par le fait qu'elle est constituée d'une dispersion de vésicules de lipides amphiphiles ioniques ou non-ioniques délimitées par un ou plusieurs feuillet(s) lipidique(s) dans une phase aqueuse de dispersion physiologiquement acceptable D, le(s) dérivé(s) rétinoïde(s) se trouvant dans les feuillets lipidiques des vésicules alors que le(s) dérivé(s) de pyrimidine est (ou sont) sous forme de solution ou, partiellement ou totalement, sous forme de suspension dans la phase aqueuse D.

La composition selon l'invention a l'avantage d'être stable à la lumière. Il n'est pas nécessaire de la stocker dans un flacon teinté ou parfaitement opaque. Par ailleurs, il faut remarquer que la composition ne contient pas d'autre solvant que l'eau : elle n'est donc pas irritante. Au contraire, les lipides ioniques ou non-ioniques formant les vésicules ont une action adoucissante vis-à-vis du cuir chevelu. De plus, elle permet une absorption

cutanée optimum et une bonne distribution des substances actives, avec une libération progressive et contrôlée des actifs qui assure une action prolongée entre deux applications.

Les vésicules de lipides amphiphiles non-ioniques et les vésicules de lipides ioniques sont, de façon connue, des vésicules délimitées par un ou plusieurs feuillets bimoléculaires ou multimoléculaires de lipide encapsulant une phase aqueuse E ; elles sont dispersées dans une phase aqueuse de dispersion D. Les vésicules de lipides amphiphiles ioniques sont, par exemple, décrites dans l'article de A.D. BANGHAM publié dans J. Mol. Biol. (1965), 13, 238-252 et les vésicules de lipides non-ioniques dans le brevet français 2 315 991.

Parmi les lipides ioniques utilisables pour obtenir des vésicules, on peut citer les phospholipides naturels ou synthétiques. On peut, en particulier, utiliser la lécithine de soja ou la lécithine hydrogénée.

Les lipides non-ioniques utilisés pour la fabrication des vésicules sont avantageusement choisis dans le groupe formé par les composés de formule (I) :

$$RO-\left[C_3H_5(OH)O\right]_{\bar{n}}-H \qquad (I)$$

où $-C_3H_5(OH)O-$ est représenté par les structures suivantes, prises en mélange ou séparément :

- $CH_2CHOHCH_2O-$ ;

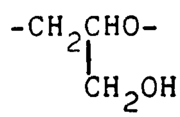 ; 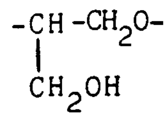

- où $\bar{n}$ est une valeur statistique moyenne comprise entre 2 et 6 ;
- où R est :

a) soit une chaîne aliphatique $R_1$ ou un reste $R_2CO$, $R_1$ étant un radical aliphatique, linéaire ou ramifié, en $C_{12}$-$C_{18}$ et $R_2$ étant un radical aliphatique, linéaire ou ramifié,en $C_{11}$-$C_{17}$ ;

b) soit $R_3\{O - C_2H_3(R_4)\}$ où $- OC_2H_3(R_4)-$ est représenté par les structures suivantes, prises en mélange ou séparément :

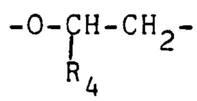 et 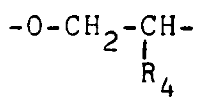 ;

$R_3$ étant un radical $R_1$ ou $R_2CO$ et

$R_4$ étant un radical $R_1$ et $R_1$ et $R_2$ ayant les significations définies ci-dessus ;

et les phytostérols oxyéthylénés.

De façon connue, le(s) lipide(s) amphiphile(s) formant les vésicules peu(ven)t être associé(s) à divers additifs modifiant la perméabilité ou la charge superficielle des vésicules. Parmi ces additifs, on peut citer, entre autres, les stérols et leurs dérivés et les esters phosphoriques d'alcools gras.

Les vésicules de lipide(s) amphiphile(s) ont des dimensions généralement comprises entre 10 nm et 5.000 nm.

Le poids total de lipide(s) formant les vésicules, y compris éventuellement le (ou les) additif(s) associé(s) représente entre 0,2 et 20% du poids total de la composition et de préférence entre 0,5 et 8%.

Le dérivé de pyrimidine utilisé selon l'invention est, de préférence, un dérivé de formule

6

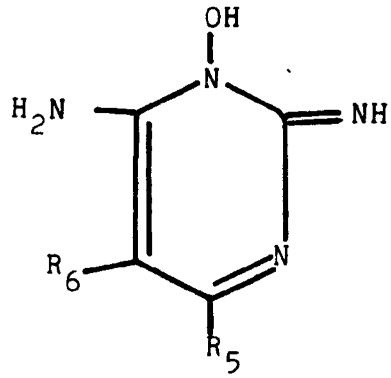

(II)

formule dans laquelle :

- $R_5$ représente un groupement

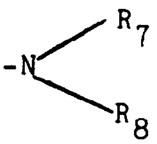

dans lequel $R_7$ et $R_8$ sont choisis parmi l'hydrogène, un groupement alkyle en $C_1$-$C_4$, alcényle en $C_2$-$C_5$, (alkyl en $C_1$-$C_4$)aryle, et cycloalkyle en $C_4$-$C_7$ ou forment un hétérocycle avec l'atome d'azote auquel ils sont liés choisi parmi les groupements aziridinyle, azétidinyle, pyrrolidinyle, pipéridinyle, hexahydroazépinyle, heptaméthylèneimine, octaméthylèneimine, morpholine ou (alkyl en $C_1$-$C_4$)-4-pipérazinyle, les groupements hétérocycliques pouvant être substitués sur les atomes de carbone par un à trois groupements alkyle en $C_1$-$C_4$, hydroxy ou alcoxy en $C_1$-$C_4$;

- $R_6$ représente l'hydrogène, un groupement alkyle en $C_1$-$C_4$, alcényle en $C_2$-$C_5$, cycloalkyle en $C_4$-$C_7$, aryle, (alkyl en $C_1$-$C_4$)aryle, aryl(alkyle en $C_1$-$C_4$), (alkyle en $C_1$-$C_4$)aryl(alkyle en $C_1$-$C_4$),alcoxy(alkyle en $C_1$-$C_4$) (alcoxy en $C_1$-$C_4$)aryl (alkyle en $C_1$-$C_4$) ou haloaryl(alkyle en $C_1$-$C_4$ )et/ou au moins un sel d'addition d'acides physiologiquement acceptables.

Les composés préférés sont constitués par des composes de formule (II) dans laquelle $R_6$ désigne l'hydrogène et $R_5$ représente un groupement

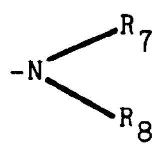

,

groupement dans lequel $R_7$ et $R_8$ forment un cycle pipéridinyle ainsi que leurs sels tels que, par exemple, le sulfate. Parmi ces composés, le composé particulièrement préféré est constitué par l'amino-6 dihydro-1,2 hydroxy-1 imino-2 pipéridino-4 pyrimidine généralement appelée minoxidil.

Le (ou les) dérivé(s) de pyrimidine peut (ou peuvent) être présent(s) dans la phase aqueuse D de dispersion sous forme de solution. Il(s) peu(ven)t être ègalement, au moins partiellement, en suspension dans la phase aqueuse D. Dans ce dernier cas, le dérivé est avantageusement sous forme de particules ayant une granulométrie inférieure à 80 µm, de préférence inférieure à 20 µm et, mieux encore, inférieure à 5 µm.

Le (ou les) dérivé(s) de pyrimidine est (ou sont) présent(s) dans la composition dans des proportions comprises, de préférence, entre 0,05 et 6%, plus particulièrement, entre 0,10 et 5 % et mieux encore entre 0,1 et 2 % en poids par rapport au poids total de la composition.

Le dérivé rétinoïde est, de préférence, un dérivé de formule (III) :

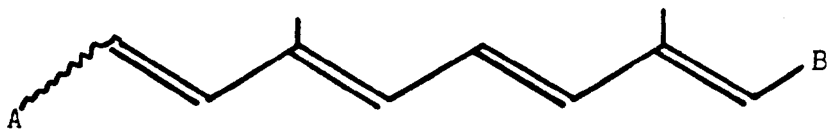

(III)

formule dans laquelle :

a) ou bien A est un groupement choisi parmi les groupements de formules :

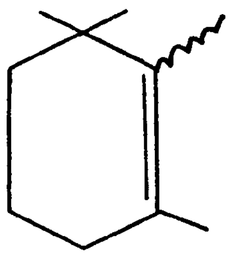

(IVa)

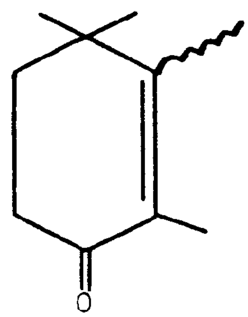

(V)

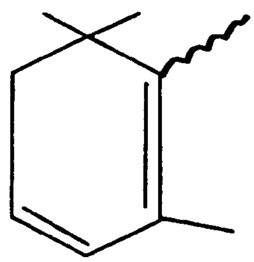

(IVb)

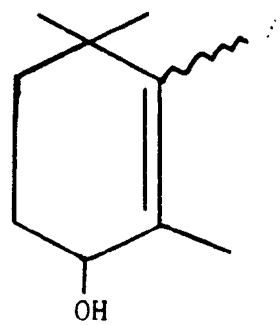

(VI)

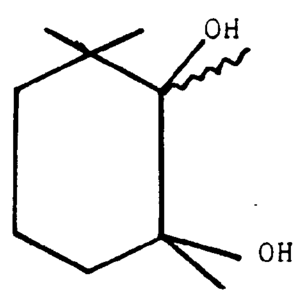

(VII)

B étant alors choisi :

aa) A est un groupement de formule IVa :

- parmi les groupements suivants :

. -CHO ;

. $-CH_2OR_9$, dans lequel - $R_9$ est l'hydrogène ou un radical alkyle en $C_1$-$C_4$ ;

.

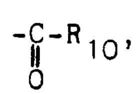

où $R_{10}$ est un radical alkyle linéaire ou ramifié en $C_1$-$C_{16}$ ;

. $-CH_2SR_{11}$, dans lequel $R_{11}$ est l'hydrogène ou un radical méthyle ;

.

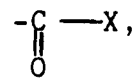

dans lequel X désigne :

(i) -OH ;

(ii) $-OR_{12}$ où $R_{12}$ est un radical alkyle en $C_1$-$C_{15}$, aryl(alkyle en $C_1$-$C_4$), substitué ou non sur le groupement aryle, arylcarboxy(alkyle en $C_1$-$C_4$), substitué ou non sur le groupement aryle, hydroxy(alkyle en $C_1$-$C_4$), amido(alkyle en $C_1$-$C_4$) ;

(iii) $-NR_{13}R_{14}$, dans lequel $R_{13}$ ou $R_{14}$, identiques ou différents, désignent l'hydrogène, un radical alkyle en $C_1$-$C_6$, hydroxyalkyle en $C_1$-$C_4$, aryle substitué ou non, un hétérocycle substitué ou non ou dans lequel $R_{13}$ et $R_{14}$ forment, conjointement avec l'atome d'azote auquel ils sont reliés, un hétérocycle substitué ou non ;

(iv) le groupement $N_3$ ;

. $-CH_2NHR_{15}$, où $R_{15}$ désigne un radical benzoyle substitué ou non ;

ab) si A est un groupement de formule IVb, V, VI ou VII : parmi -COOH et les formes salifiées ou estérifiées correspondantes ;

b) ou bien A est choisi dans le groupe formé par les groupements aryle, substitués ou non, les hétérocycles, substitués ou non, les groupements arylhétérocycliques, substitués ou non sur l'hétérocycle, ou les groupements arylhomocycliques, substitués ou non sur le noyau aromatique,

B étant alors choisi parmi les groupements : -COOH, $-COOR_{16}$ où $R_{16}$ est un radical alkyle en $C_1$-$C_4$ ou un radical amide substitué par un alkyle en $C_1$-$C_4$, ainsi que leurs sels et leurs esters physiologiquement acceptables.

Les dérivés de formule (III) sont, de préférence, ceux dans lesquels (alkyle $C_1$-$C_4$) désigne méthyle, éthyle, n-butyle, t-butyle ; (alkyle $C_1$-$C_{16}$) désigne éthyle, propyle, palmityle ; aryle désigne phényle ou benzyle, les substituants des groupements aryle étant (alkyle $C_1$-$C_4$), (alcoxy $C_1$-$C_{12}$), hydroxyle, halogène, nitro, les substituants alcoxy ou alkyle pouvant eux-mêmes être substitués par un groupement OH.

Les groupements hétérocycliques définis dans la formule (III) au paragraphe [aa)iii)] peuvent être, entre autres, des groupements dérivés de la phtalimide et de la succinimide, des hétérocycles de 4 à 6 chaînons comportant un ou plusieurs atomes d'oxygène et/ou d'azote.

Le dérivé de la famille des rétinoïdes de formule (III) défini ci-dessus au paragraphe a) est, en particulier, choisi parmi le rétinal, le rétinol; l'acétate, le propionate et le palmitate de rétinyle; l'acide rétinoïque sous les formes tout-trans, 13-cis, 9-cis, 11-cis, 9, 13-dicis, 11,13-dicis; les rétinoates de zinc correspondants; les rétinoates d'ammonium quaternaire de formule :

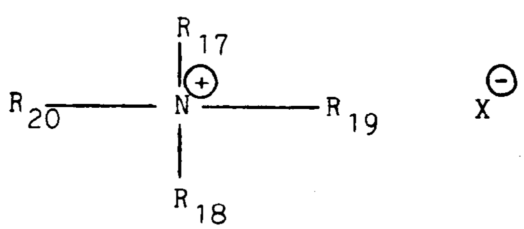

(VIII)

formule dans laquelle :

$X^{\ominus}$ désigne un radical rétinoate tout-trans ou 13-cis, et

i) ou bien $R_{17}$, $R_{18}$, $R_{19}$, identiques ou différents, sont un radical alkyle linéaire en $C_1$-$C_4$, substitué ou non par un ou plusieurs hydroxyle, $R_{20}$ étant un radical alkyle ou alcényle linéaire en $C_{12}$-$C_{18}$ ;

ii) ou bien $R_{19}$ est un groupement

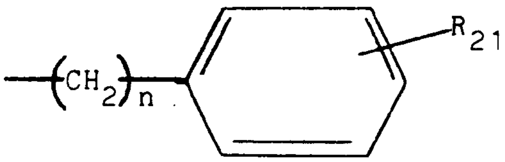

dans lequel :

n est égal à 0 ou 1,

$R_{21}$ représente un atome d'hydrogène, ou halogène, un groupement hydroxyle, alkyle ou hydroxyalkyle en $C_1$-$C_{18}$ ou acyle en $C_2$-$C_{18}$ ;

$R_{17}$, $R_{18}$ et $R_{19}$ ayant les significations indiquées sous i) ;

iii) ou bien $R_{17}$ et $R_{18}$ forment un hétérocycle aliphatique comportant au moins un atome d'oxygène, d'azote ou de soufre,

$R_{19}$ et $R_{20}$ ayant les significations indiquées sous i) et ii)

D'autres composés rentrant dans la définition des rétinoïdes de la formule (III) définis ci-dessus au paragraphe a), particulièrement utilisables conformément à l'invention, sont choisis parmi le tout-trans rétinoyloxyacétamide; le mélange des rétinoates tout-trans de 2-hydroxy 1-propyle et de 1-hydroxy 2-propyle; le 2-hydroxyéthyl tout-trans rétinoate; le 4-nitrobenzyl tout-trans rétinoate; le benzyl tout-trans rétinoate; le 4-(tout-trans rétinoyl oxyacétyl) catéchol; le 2-cyclohexyléthyl tout-trans rétinoate; le 10-carboxyméthyldécyl tout-trans rétinoate; le 4-hydroxybutyl tout-trans rétinoate; le cholestéryl tout-trans rétinoate; le 4-bromobenzyl tout-trans rétinoate; le cholestéryl tout-trans rétinoyloxyacétate; le tout-trans rétinoyloxyacétyl benzène; le 4-(tout-trans rétinoyloxyacétyl)-bromo benzène; le 4-(tout-trans rétinoyloxyacétyl)-nitrobenzène; le 4-(tout-trans rétinoyloxyacétyl)-benzonitrile; le tout-trans rétinoyloxyacétyl-2,4 dichlorobenzène; le N-(tout-trans rétinoyloxy)phtalimide; le N-(tout-trans rétinoyloxy)succinimide; le 4-(tout-trans rétinoyloxyacétyl)-méthoxybenzène; le 4-(tout-trans rétinoyloxyacétyl)-phénol; le 1-(tout-trans rétinoyloxyacétyl)3,4,5-trimethoxybenzène; le 1-(tout-trans rétinoyloxyacétyl)2,4,6-triméthylbenzène; le 4-(tout-trans rétinoyloxyacétyl) toluène; le 4-(tout-trans rétinoyloxyacétyl) éthoxybenzène; le 4-(tout-trans rétinoyloxyacétyl) acétoxybenzène; le 4-(tout-trans rétinoyloxyacétyl) naphtalène; le 4-(tout-trans rétinoyloxyacétyl)biphényle; le 4-(tout-trans rétinoyloxyacétyl) 2,5-diméthoxybenzène; le 1-(tout-trans rétinoyloxyacétyl)2,4-diméthylbenzène; le 1-(tout-trans rétinoyloxyacétyl)3,4-diacétoxybenzène; le tout-trans rétinamide; le 2-hydroxyéthyl tout-trans rétinamide; le N-éthyl tout-trans rétinamide; le 4-(tout-trans rétinoyl) aminophénol; le N-méthyldiméthyldioxolane rétinamide; le N-(orthocarboxyphényl) rétinamide; le N-(p-carboxyphényl) rétinamide; le N-hydroxypropyl tout-trans rétinamide; le N-(hydroxypropyl) 13-cis rétinamide; le N-(5-tétrazolyl) tout-trans rétinamide; le N-(5-tétrazolyl) 13-cis rétinamide; le N-(3,4 méthylène dioxyphénylméthyl) tout-trans rétinamide; le N-(n-propyl) tout-trans rétinamide; le N-tertiobutyl tout-trans rétinamide; le N-(1,1,3,3 tétraméthyl) butyl tout-trans rétinamide; le N-(4 carboxyméthyl 3-hydroxyphényl) tout trans rétinamide; le N-[β -(3,4 diméthoxyphényl) éthyl] tout- trans rétinamide; le 2-(tout-trans rétinoylamino) benzotriazole; le 1-(tout-trans rétinoyl) 1,2,4 triazole; le N-(tout-trans rétinoyl)imidazole; la 1-nicotinoyl 2-(tout-trans rétinoyl) hydrazine; la N-(tout-trans rétinoyl) morpholine; la trans-β-ionone (tout-trans rétinoyl) hydrazone; la N,N'-dicyclohexyl N-(tout-trans rétinoyl) urée; l'acétone (tout-trans rétinoyl) hydrazone; la N-benzoylrétinylamine; l'azoture de rétinoyle.

Les groupements représentés par A dans la formule III et définis ci-dessus dans le paragraphe b) en liaison avec les groupements aryle, aryle substitué, hétérocycle, hétérocycle substitué, aryl-hétérocycle substitué sur l'hétérocycle ou aryl-homocycle substitué sur le noyau aromatique, sont, en particulier, choisis parmi les groupements suivants :

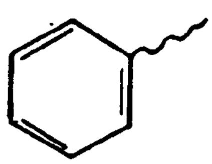

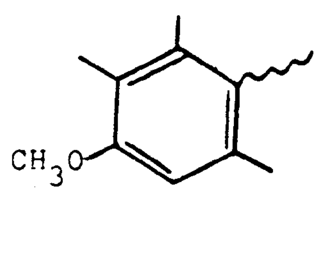

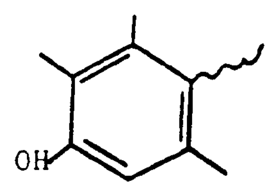

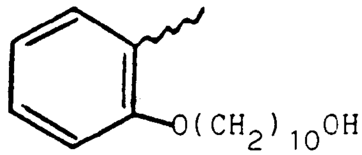

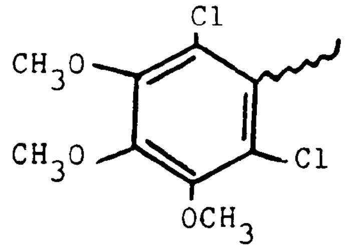

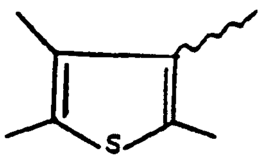

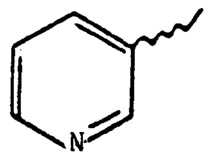

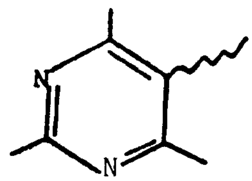

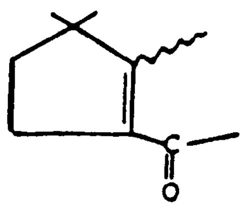

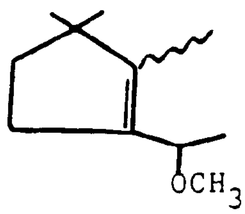

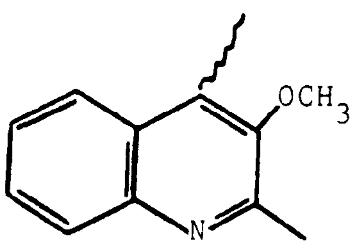

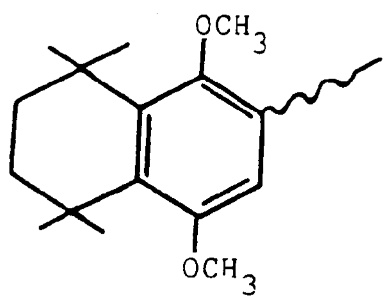

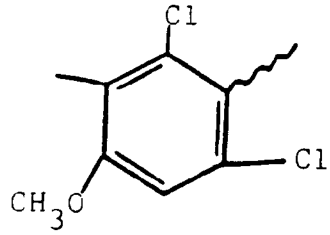

le groupement B étant alors COOH, $CONHC_2H_5$, $COOC_2H_5$.

Les composés particulièrement préférés dans cette famille sont le motrétinide et l'étrétinate.

D'autres rétinoïdes utilisables conformément à l'invention sont ceux répondant aux formules (IX) à (XIV) suivantes, ainsi que leurs sels ou esters physiologiquement acceptables :

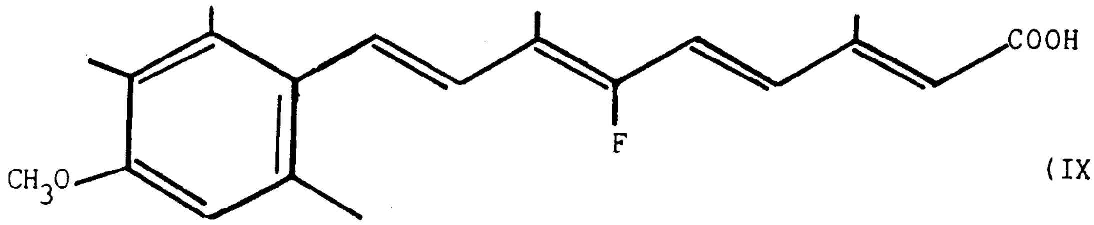

(IX)

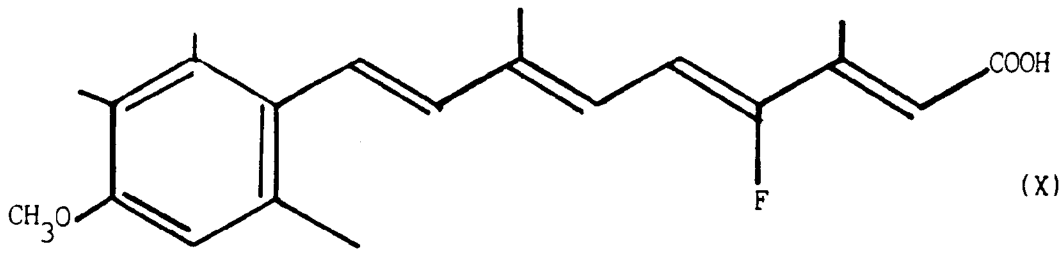

(X)

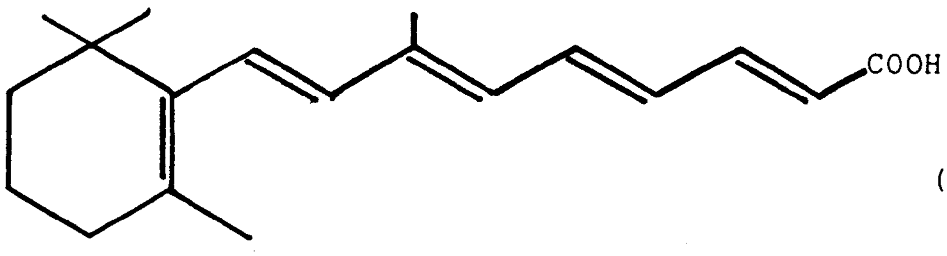

(XI)

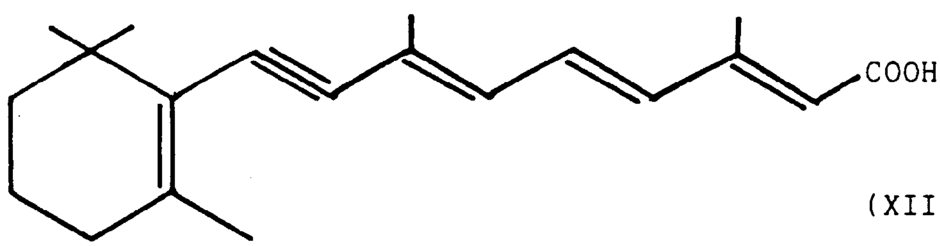

(XII)

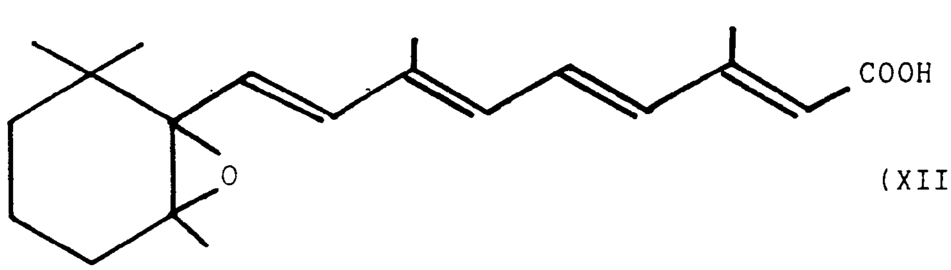

(XIII)

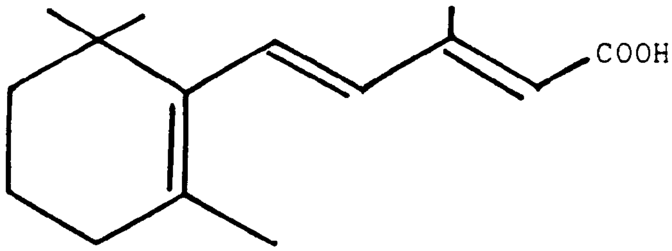

(XIV)

Des composés de la famille des rétinoïdes utilisables conformément à l'invention, sont également décrits dans les brevets US-A 4 190 594, 4 126 698, EP-A 010 209, EP-A 010 208, EP-A 097 76, FR-A 2 293 193 et EP-A 033 095

Dans la composition selon l'invention, parmi les rétinoïdes de formule (III) , tels que définis aux paragraphes a) et b), on préfère plus particulièrement ceux répondant à la formule générale suivante :

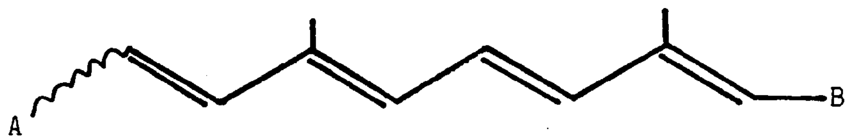

(IV)

qui sont sous forme d'isomères tout-trans ou 13-cis et correspondent à une formule dans laquelle :

- B représente :
  . ou bien un groupement

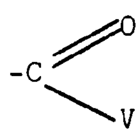

  dans lequel V est OH; OZ, Z désignant un groupement alkyle en $C_1$-$C_{15}$ ; ou un radical amino non substitué ou mono- ou di- substitué par un alkyle en $C_1$-$C_6$ ;
  . ou bien un groupement $-CH_2OH$ ou -CHO ;
- A représente un groupement

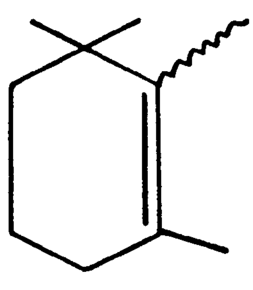

ou

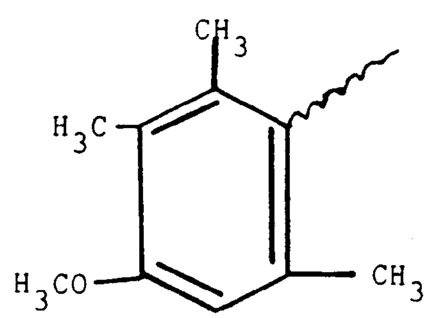

Parmi les dérivés particulièrement préférés, on peut citer les produits dénommés couramment : isotrétinoïne; rétinol; motrétinide; étrétinate; acétate, palmitate et propionate de rétinyle; tout-trans rétinoate de zinc ; et plus particulièrement encore trétinoïne ou acide tout-trans rétinoïque.

La composition selon l'invention contient, de préférence, du minoxidil en association avec de l'acide tout-trans rétinoïque.

Dans la composition selon l'invention, le dérivé rétinoïde de formule III dans laquelle B est -COOH, est présent dans des proportions comprises entre 0,005 % et 0,030 % en poids par rapport au poids total de la composition.

La phase aqueuse D peut être gélifiée. Dans ce cas, on utilise des agents épaississants ou gélifiants bien connus dans l'état de la technique, tels que, plus particulièrement, les hétérobiopolysaccharides comme la gomme de xanthane ou les scléroglucanes, les dérivés de cellulose, les polymères acryliques réticulés ou non.

Les gélifiants ou épaississants sont présents, de préférence, dans des proportions comprises entre 0,1 et 5 %, en particulier, entre 0,4 et 3 % en poids par rapport au poids total de la composition.

La phase aqueuse D peut également contenir des additifs hydrosolubles ou dispersés, physiologiquement acceptables. Ces additifs sont plus particulièrement ceux utilisés dans les compositions pour application topique, cosmétiques ou pharmaceutiques, tels que par exemple, des agents conservateurs, des colorants, des agents alcalinisants ou acidifiants.

Le pH des compositions selon l'invention est, de préférence, compris entre 4 et 9.

La composition selon la présente invention peut être préparée par tout procédé connu de fabrication de vésicules des lipides ioniques ou non-ioniques, dans lequel on introduit le dérivé rétinoïde en l'ajoutant au mélange lipidique avant d'effectuer l'hydratation dudit mélange lipidique et de former les vésicules, et dans lequel on introduit le dérivé de pyrimidine dans la phase aqueuse de dispersion des vésicules, après formation des vésicules.

La présente invention a aussi pour objet la composition selon l'invention pour utilisation comme médicament destiné à induire et stimuler la croissance des cheveux et diminuer leur chute.

La présente invention a enfin pour objet un procédé de traitement des cheveux et du cuir chevelu, caractérisé par le fait qu'on leur applique 1 à 5 g de la composition selon l'invention, à une fréquence de une à deux applications par jour pendant 1 à 7 jours par semaine, pendant un temps de traitement compris entre 1 et 6 mois. L'application est, de préférence effectuée sur la zone alopécique.

Le procédé de traitement présente les caractéristiques d'un traitement thérapeutique dans la mesure où l'association conforme à l'invention a une activité thérapeutique au niveau des mécanismes biologiques du cycle pilaire et du dysfonctionnement de celui-ci. Ce procédé est également un traitement cosmétique dans la mesure où il permet d'améliorer l'esthétique des cheveux en leur donnant une plus grande vigueur et un aspect plus satisfaisant en l'absence de toute pathologie particulière.

Les exemples donnés ci-après, à titre purement illustratif et non limitatif, permettront de mieux comprendre l'invention.

<u>EXEMPLE 1</u> (Comparatif)

1) Préparation des compositions

On a préparé, selon le procédé décrit dans l'exemple 2 donné ci-après, une composition $A_1$ correspondant à l'invention et contenant 0,025 % en poids, par rapport au poids total de la composition, d'acide tout-trans rétinoïque dans les feuillets lipidiques de vésicules de lipides non-ioniques et 0,2 % en poids de minoxidil dans de l'eau.

On a également préparé une composition $B_1$ (ne faisant pas partie de l'invention) contenant 0,025 % en poids d'acide tout-trans rétinoïque et 0,2 % en poids de minoxidil dans un solvant constitué (en volume) de 5% de propylèneglycol et 95 % d'éthanol.

2) Essais comparatifs

On a stocké les deux compositions $A_1$ et $B_1$ dans des flacons en verre transparent, à des températures variables. On a mesuré au bout de 15 jours la concentration en acide tout-trans rétinoïque et on a calculé le pourcentage d'acide restant et la perte en acide. Les résultats obtenus sont donnés dans le tableau I ci-après.

TABLEAU I

| Température | Composition $A_1$ | | Composition $B_1$ | |
|---|---|---|---|---|
| | % acide restant | Perte en % | % acide restant | Perte en % |
| 4°C | 97,4 | 2,6 | 91,7 | 8,3 |
| Ambiante | 95 | 5 | 90,7 | 9,3 |
| 45°C | 93,3 | 6,7 | 82,3 | 17,7 |

On voit que l'acide rétinoïque est beaucoup mieux préservé dans une composition selon l'invention que dans une composition selon l'état de la technique.

EXEMPLE 2

Pour préparer des niosomes contenant de l'acide tout-trans rétinoïque dans les feuillets lipidiques, on charge une cuve munie d'une agitation raclante à la température de 35°C successivement avec les composés suivants:

- Lipide non-ionique de formule : $C_{16}H_{33}-[OCH_2-CHOH-CH_2]_2OH$ 912 g
- Cholestérol.............................. 912 g
- Dicétylphosphate.......................... 96 g
- Chlorure de méthylène..................... 10880 g
- Acide tout-trans rétinoïque (en solution dans 220 g de chlorure de méthylène)(matière active) 6 g
- Sel disodique de l'acide éthylène diaminotétracétique.............................. 12 g
- Eau....................................... 23400 g

Après distillation du chlorure de méthylène sous pression réduite (80-500 x $10^2$ Pascal), on élève la température de la cuve à 52°C et on distille l'eau sous une pression réduite de 930 x $10^2$ Pascal.

On obtient 12 kilogrammes de vésicules titrant 0,044 % en poids d'acide tout-trans rétinoïque.

On disperse, avec un ultradisperseur "Virtis", les vésicules obtenues dans une solution aqueuse de minoxidil et on obtient la formulation $A_1$ suivante :

| | | | |
|---|---|---|---|
| - Minoxidil............................... | | 0,2 | g |
| - Vésicules correspondant à 0,025 g d'acide tout-trans rétinoïque.................. | | 57 | g |
| - Eau................................ | qsp | 100 | ml |

On a utilisé cette composition à raison de deux applications topiques de 3 g par jour, sur les cheveux et le cuir chevelu d'un sujet atteint d'alopécie, tous les jours pendant 3 mois. On a constaté une repousse significative des cheveux.

EXEMPLE 3

On prépare la formulation suivante :

| | | | |
|---|---|---|---|
| - Minoxidil............................... | | 1 | g |
| - Vésicules de l'exemple 2................ | | 57 | g |
| - Acide polyacrylique réticulé (PM = 3 millions environ) vendu sous la dénomination commerciale "CARBOPOL 934" par la société "GOODRICH"...................... | | 0,5 | g |
| - Eau................................ | qsp | 100 | ml |

La composition se présente sous forme d'une crème.

On a utilisé cette composition à raison d'une application topique de 5 g par jour sur les cheveux et le cuir chevelu d'un sujet atteint d'alopécie tous les jours pendant 5 mois. On a constaté une repousse significative des cheveux.

EXEMPLE 4

On charge une cuve munie d'une agitation raclante, à la température de 35°C, successivement avec les composés suivants :

| | | |
|---|---|---|
| - Lipide non-ionique de formule : $C_{16}H_{33}-[OCH_2-CHOH-CH_2]_2-OH$ ..... | 380 | g |
| - Cholestérol ............................ | 380 | g |
| - Dicétylphosphate ....................... | 40 | g |
| - Chlorure de méthylène .................. | 3200 | g |
| - Acide tout-trans rétinoïque.............. | 10 | g |
| - Chlorure de méthylène solubilisant l'acide tout-trans rétinoïque............ | 800 | g |
| - DL α-tocophérol ....................... | 10 | g |
| - Sel disodique de l'acide éthylène diamino tétracétique .................. | 10 | g |
| - Eau .................................... | 13790 | g |

Après distillation du chlorure de méthylène sous pression réduite on élève la température de la cuve à 50°C et on distille l'eau sous pression réduite.

On obtient 10 kilogrammes de vésicules titrant 0,099 % en poids d'acide tout-trans rétinoïque.

On disperse, avec un ultradisperseur "VIRTIS", les vésicules obtenues dans une solution aqueuse de minoxidil et on obtient la formulation $A_2$ suivante :

| | | |
|---|---|---|
| - Minoxidil .............................. | 0,2 | g |
| - Vésicules correspondant à 0,025 g d'acide tout-trans rétinoïque .................... | 25,3 | g |
| - DL α- tocophérol ......................... | 0,025 | g |
| - Eau ...........................qsp........ | 100 | ml |

On a utilisé cette composition à raison d'une application topique de 5 g par jour sur les cheveux et le cuir chevelu d'un sujet atteint d'alopécie, tous les jours pendant 5 mois. On a constaté une repousse significative des cheveux.

On a également préparé une composition $B_2$ de référence (ne faisant pas partie de l'invention) ayant la formulation suivante :

| | | |
|---|---|---|
| - Minoxidil ........................... | 0,2 | g |
| - Acide tout-trans rétinoïque .......... | 0,025 | g |
| - DL α - tocophérol ................... | 0,025 | g |
| - Propylèneglycol/Alcool éthylique (5/95 en volume) ..................qsp | 100 | ml |

EXEMPLE 5

On charge une cuve munie d'une agitation raclante, à la température de 35°C, successivement avec les composés suivants :

- Lipide non-ionique de formule :

$$C_{12}H_{25}-\left[O\ C_2H_3\ (R)\right]-O-\left[C_3H_5(OH)-O\right]_{\bar{n}}-H$$

où -O $C_2H_3$(R)- est constitué par un mélange des radicaux :

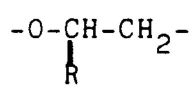 et 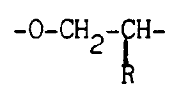 ;

où

-$C_3H_5$(OH)-O- est constitué par un mélange des radicaux :

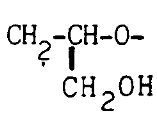 et 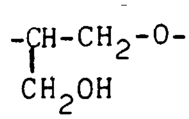 ;

où $\bar{n}$ = 6 ;

| | | |
|---|---|---|
| et où R est un mélange des radicaux $C_{14}H_{29}$ et $C_{16}H_{33}$ | 380 | g |
| - Cholestérol | 380 | g |
| - Chlorure de méthylène | 3200 | g |
| - Acide tout-trans rétinoïque | 10 | g |
| - Chlorure de méthylène solubilisant l'acide tout-trans rétinoïque | 800 | g |
| - DL α-tocophérol | 10 | g |
| - Sel disodique de l'acide éthylène-diaminotétracétique | 10 | g |
| - Eau | 13855 | g |

Après distillation du chlorure de méthylène sous pression réduite, on élève la température de la cuve à 50°C et on distille l'eau sous pression réduite.

On obtient 10 kg de vésicules titrant 0,099 % en poids d'acide tout-trans rétinoïque.

On disperse, avec un ultradisperseur "VIRTIS", les vésicules obtenues dans une solution aqueuse de minoxidil et on obtient la formulation $A_3$ suivante :

| | | |
|---|---|---|
| - Minoxidil | 0,2 | g |
| - Vésicules correspondant à 0,025 g d'acide tout-trans rétinoïque | 25,2 | g |
| - DL α-tocophérol | 0,025 | g |
| - Eau qsp | 100 | ml |

On a utilisé cette composition à raison d'une application topique de 5 g par jour sur les cheveux et le cuir chevelu d'un sujet atteint d'alopécie, tous les jours pendant 5 mois. On a constaté une repousse significative des cheveux.

On a également préparé une composition $B_3$ de référence (ne faisant pas partie de l'invention), identique à la composition $B_2$ de l'exemple 4.

EXEMPLE 6

On charge une cuve munie d'une agitation raclante, à la température de 35°C, successivement avec les composés suivants :

- Lipide non-ionique de formule :

$$C_{15}H_{31} - CO - [OCH_2 - CHOH - CH_2]_2 - OH \quad 380 \text{ g}$$

| | | |
|---|---|---|
| - Cholestérol ........................ | 380 | g |
| - Dicétylphosphate .................... | 40 | g |
| - Chlorure de méthylène ................ | 3280 | g |
| - Acide tout-trans rétinoïque ........... | 10 | g |
| - Chlorure de méthylène solubilisant l'acide tout-trans rétinoïque........... | 800 | g |
| - DL α-tocophérol ..................... | 10 | g |
| - Sel disodique de l'acide éthylène diaminotétracétique .................. | 10 | g |
| - Eau ................................ | 13800 | g |

Après distillation du chlorure de méthylène sous pression réduite, on élève la température de la cuve à 50°C et on distille l'eau sous pression réduite.

On obtient 10 kg de vésicules titrant 0,099 % en poids d'acide tout-trans rétinoïque.

On disperse, avec un ultradisperseur "VIRTIS", les vésicules obtenues dans une solution aqueuse de minoxidil et on obtient la formulation $A_4$ suivante :

| | | |
|---|---|---|
| - Minoxidil ........................ | 0,2 | g |
| - Vésicules correspondant à 0,025 g d'acide tout-trans rétinoïque ..... | 25,4 | g |
| - DL α-tocophérol ................. | 0,025 | g |
| - Eau ......................... qsp | 100 | ml |

On a utilisé cette composition à raison d'une application topique de 5 g par jour sur les cheveux et le cuir chevelu d'un sujet atteint d'alopécie, tous les jours pendant 5 mois. On a constaté une repousse significative des cheveux.

On a également préparé une composition $B_4$ de référence (ne faisant pas partie de l'invention), identique à la composition $B_2$ de l'exemple 4.

EXEMPLE 7

On charge une cuve munie d'une agitation raclante à la température de 38°C successivement en deux étapes.

Dans une première étape, on a ajouté les composés suivants :

| | | |
|---|---|---|
| - Eau............................. | 10320 | g |
| - Sel disodique de l'acide éthylène diaminotétracétique................ | 10 | g |

Dans une seconde étape, on a ajouté les composés suivants :

| | | |
|---|---|---|
| - Lécithine de soja (phosphatidyl choline 75%) vendu par la Société "SEPPIC" sous la dénomination commerciale "LIPOID S 75" | 600 | g |
| - Butylhydroxytoluène................ | 7,5 | g |
| - DL α -tocophérol.................. | 7,5 | g |
| - Acide tout-trans rétinoïque........ | 7,5 | g |
| - Chlorure de méthylène.............. | 2400 | g |

Après distillation du chlorure de méthylène sous pression réduite, on élève la température de la cuve à 50°C et on distille l'eau sous pression réduite.

On obtient 7,5 kg de liposomes titrant 0,102 % d'acide tout-trans rétinoïque.

On disperse, avec un ultradisperseur "VIRTIS", les liposomes obtenus dans une solution aqueuse de minoxidil et on obtient la formulation $A_5$ suivante :

| | | |
|---|---|---|
| - Minoxidil ......................... | 0,2 | g |
| - Liposomes correspondant à 0,025 g d'acide tout-trans rétinoïque ...... | 24,5 | g |
| - DL α-tocophérol .................. | 0,025 | g |
| - Butylhydroxytoluène ............... | 0,025 | g |
| - Eau ....................... qsp .. | 100 | ml |

On a utilisé cette composition à raison d'une application topique de 5 g par jour sur les cheveux et le cuir chevelu d'un sujet atteint d'alopécie, tous les jours pendant 5 mois. On a constaté une repousse significative des cheveux.

On a également préparé une composition $B_5$ de référence (ne faisant pas partie de l'invention) ayant la formulation suivante :

| | | |
|---|---|---|
| -Minoxidil ........................ | 0,2 | g |
| -Acide tout-trans rétinoïque ....... | 0,025 | g |
| -Butylhydroxytoluène ............... | 0,025 | g |
| -DL α- tocophérol | 0,025 | g |
| -Propylèneglycol/Alcool éthylique (5/95 en volume) ......... qsp .... | 100 | ml |

EXEMPLE 8

On charge une cuve munie d'une agitation raclante à la température de 38°C successivement en trois étapes.

Dans une première étape, on a ajouté les composés suivants :

| | | |
|---|---|---|
| - Eau ............................ | 13800 | g |
| - Sel disodique de l'acide éthylène-diaminotétracétique................ | 10 | g |

Dans une deuxième étape, on a ajouté les composés suivants :

| | | |
|---|---|---|
| - Lécithine hydrogénée à 30/35 % de phosphatidylcholine hydrogéné vendu par la Société "NIKKO" sous la dénomination "LECINOL S 10".............. | 480 | g |
| - Phytostérol polyoxyéthylené (à 5 moles d'oxyde d'éthylène) vendu par la Société "NIKKO" sous la dénomination "GENEROL 122 E 5" | 320 | g |
| - Chlorure de méthylène (solubilisant le "LECINOL" et le "GENEROL")......... | 3200 | g |

Dans une troisième étape, on a ajouté les composés suivants :

| | | |
|---|---|---|
| - Acide tout-trans rétinoïque.......... | 10 | g |
| — DL α -tocophérol.................... | 10 | g |
| - Chlorure de méthylène (solubilisant le DL α -tocophérol).................. | 800 | g |

Après distillation du chlorure de méthylène sous pression réduite, on élève la température de la cuve à 50°C et on distille l'eau sous pression réduite.

On obtient 10 kg de liposomes titrant 0,05 % en poids d'acide tout-trans rétinoïque.

On disperse, avec un ultradisperseur "VIRTIS", les vésicules obtenues dans une solution aqueuse de minoxidil et on obtient la formulation $A_6$ suivante :

| | | |
|---|---|---|
| - Minoxidil ........................ | 0,2 | g |
| - Liposomes correspondant à 0,025 g d'acide tout-trans rétinoïque ..... | 50 | g |
| - DL α -tocophérol .................. | 0,05 | g |
| - Eau .................. qsp ....... | 100 | ml |

On a utilisé cette composition à raison d'une application topique de 5 g par jour sur les cheveux et le cuir chevelu d'un sujet atteint d'alopécie, tous les jours pendant 5 mois. On a constaté une repousse significative des cheveux.

On a également préparé la composition $B_6$ de référence (ne faisant pas partie de l'invention) ayant la formulation suivante :

| | | |
|---|---|---|
| - Minoxidil ........................ | 0,2 | g |
| - Acide tout-trans rétinoïque ....... | 0,025 | g |
| - DL α- tocophérol ................. | 0,05 | g |
| - Propylèneglycol/Alcool éthylique (5/95 en volume) ......... qsp .... | 100 | ml |

EXEMPLE 9 : (comparatif)

On a exposé à la lumière du jour les compositions $A_2$ à $A_6$ et les compositions $B_2$ à $B_6$ correspondantes, de référence, dans des flacons en verre transparent.

On a évalué la photodégradation à la lumière du jour de l'acide tout-trans rétinoïque en présence de minoxidil dans ces compositions en mesurant la concentration en acide tout-trans rétinoïque à intervalles de temps déterminés. Les concentrations en acide tout-trans rétinoïque,en % par rapport à la concentration initiale, sont données dans le tableau II ci-après pour les différentes compositions et pour différents temps d'irradiation.

# TABLEAU II

| Temps en minutes d'irradiation / Compositions | 0 | 20 | 40 | 80 | 120 |
|---|---|---|---|---|---|
| $A_2$ | **100** | **69** | **44** | **43** | **43** |
| $A_3$ | **100** | **71** | **59,5** | **49** | **42** |
| $A_4$ | **100** | **75** | **52** | **46,5** | **41** |
| $B_2 = B_3 = B$ | **100** | **46** | **27,5** | **19** | **18** |
| $A_5$ | **100** | **62** | **54** | **35,5** | **27,5** |
| $A_6$ | **100** | **70** | **49** | **37** | **32** |
| $B_5$ | **100** | **45,5** | **35** | **20** | **17,5** |
| $B_6$ | **100** | **47,5** | **37,5** | **22,5** | **19** |

On peut voir sur le tableau II que, dans les compositions selon l'invention,l'acide tout-trans rétinoïque se

décompose beaucoup moins rapidement que dans les compositions de l'art antérieur. Par exemple, après 120 minutes d'exposition à la lumière du jour, les compositions selon l'invention $A_2$ à $A_4$ contiennent plus de 40 % d'acide tout-trans rétinoïque alors que les compositions de l'art antérieur $B_2$ à $B_4$ contiennent moins de 20 % d'acide tout-trans rétinoïque, soit plus de deux fois moins.

**Revendications pour les Etats contractants suivants : AT, BE, CH, DE, DK, FR, GB, IT, LI, NL, SE**

1. Composition cosmétique ou pharmaceutique pour application topique contenant, en association, au moins un dérivé rétinoïde et au moins un dérivé de pyrimidine, caractérisée par le fait qu'elle est constituée d'une dispersion de vésicules d'au moins un lipide amphiphile ionique et/ou non-ionique délimitées par un ou plusieurs feuillets lipidiques dans une phase aqueuse de dispersion physiologiquement acceptable D, le(s) dérivé(s) rétinoïde(s) se trouvant dans les feuillets lipidiques des vésicules, alors que le(s) dérivé(s) de pyrimidine est (ou sont) sous forme de solution ou, partiellement ou totalement, sous forme de suspension dans la phase aqueuse D.

2. Composition selon la revendication 1, caractérisée par le fait que le(s) lipide(s) amphiphile(s) constitutif(s) des feuillets des vésicules est (ou sont) un (ou des) lipide(s) ionique(s) pris dans le groupe formé par les phospholipides naturels ou synthétiques.

3. Composition selon la revendication 1, caractérisée par le fait que le(s) lipide(s) amphiphile(s) constitutif(s) des feuillets des vésicules est (ou sont) un (ou des) lipide(s) non-ionique(s) pris dans le groupe formé par les composés de formule (I) :

$$RO-\left[C_3H_5(OH)O\right]_{\bar{n}}-OH \qquad (I)$$

- où $-C_3H_5(OH)O$ est représenté par les structures suivantes prises en mélange ou séparément : $-CH_2CH_O\ CH_2O-$ ;

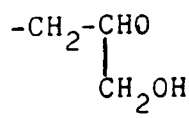 ; 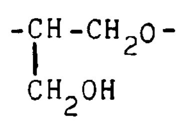

- où $\bar{n}$ est une valeur statistique moyenne comprise entre 2 et 6 ;
- ou R est :

a) soit une chaîne aliphatique $R_1$ ou un reste $R_2CO$, $R_1$ étant un radical aliphatique, linéaire ou ramifié, en $C_{12}$-$C_{18}$ et $R_2$ étant un radical aliphatique, linéaire ou ramifié, en $C_{11}$-$C_{17}$ ;
b) soit

$$R_3-\left[OC\ _2H_3(R_4)\right]-$$

où $-OC_2H_3(R_4)-$ est représenté par les structures suivantes prises en mélange ou séparément :

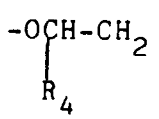 et 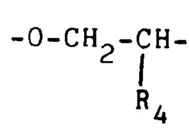

$R_3$ étant un radical $R_1$ ou $R_2CO$, $R_4$ étant un radical $R_1$, et $R_1$ et $R_2$ ayant les significations définies ci-dessus,

et les phytostérols oxyéthylénés.

**4.** Composition selon l'une des revendications 1 à 3, caractérisée par le fait que le(s) lipide(s) amphiphile(s) des feuillets est (ou sont) associé(s) à au moins un additif modifiant la perméabilité ou la charge superficielle des vésicules.

**5.** Composition selon l'une des revendications 1 à 4, caractérisée par le fait que le poids total de lipide(s) formant les vésicules, y compris éventuellement l'(ou les) additif(s) associé(s), représente entre 0,2 et 20 % du poids total de la composition.

**6.** Composition selon l'une des revendications 1 à 5, caractérisée par le fait qu'elle contient au moins un dérivé de pyrimidine de formule :

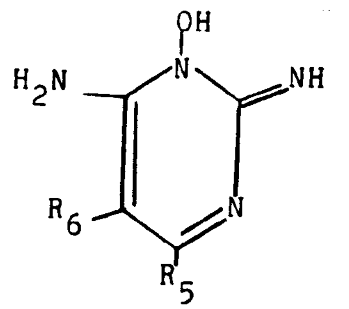

(II)

formule dans laquelle :

- $R_5$ représente un groupement

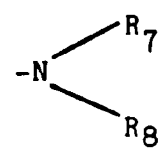

dans lequel $R_7$ et $R_5$ sont choisis parmi l'hydrogène, un groupement alkyle en $C_1$-$C_4$, alcényle en $C_2$-$C_5$, (alkyle en $C_1$-$C_4$)aryle, et cycloalkyle en $C_4$-$C_7$ ou forment un hétérocycle avec l'atome d'azote auquel ils sont liés choisi parmi les groupements aziridinyle, azétidinyle, pyrrolidinyle, pipéridinyle, hexahydroazépinyle, heptaméthylèneimine, octaméthylèneimine, morpholine ou (alkyl en $C_1$-$C_4$)-4-pipérazinyle, les groupements hétérocycliques pouvant être substitués sur les atomes de carbone par un à trois groupements alkyle en $C_1$-$C_4$,hydroxy ou alcoxy en $C_1$-$C_4$;

- $R_5$ représente l'hydrogène, un groupement alkyle en $C_1$-$C_4$, alcényle en $C_2$-$C_5$ cycloalkyle en $C_4$-$C_7$, aryle, (alkyl en $C_1$-$C_4$)aryle, aryl(alkyle en $C_1$-$C_4$), (alkyl en $C_1$-$C_4$)aryl(alkyle en $C_1$-$C_4$), alcoxy(alkyle en $C_1$-$C_4$) (alcoxy en $C_1$-$C_4$)aryl (alkyle en $C_1$-$C_4$) ou haloaryl(alkyle en $C_1$-$C_4$) et/ou au moins un sel d'addition d'acides physiologiquement acceptables.

**7.** Composition selon la revendication 6, caractérisée par le fait qu'elle contient l'amino-6 dihydro-1,2 hydroxy-1 imino-2 pipéridino-4 pyrimidine et/ou au moins un de ses sels d'addition physiologiquement acceptables.

**8.** Composition selon l'une des revendications 1 à 7, caractérisée par le fait qu'elle contient au moins un dérivé de pyrimidine, qui est, au moins partiellement, sous forme de suspension, dans la phase aqueuse D, de particules ayant une granulométrie inférieure à 80 µm.

**9.** Composition selon l'une des revendications 1 à 8, caractérisée par le fait que le(s) dérivé(s) de pyrimidine est (ou sont) présent(s) dans la composition dans des proportions comprises entre 0,05 et 6 % en poids par rapport au poids total de la composition.

**10.** Composition selon l'une des revendications 1 à 9, caractérisée par le fait que le dérivé rétinoïde est un dérivé de formule (III) :

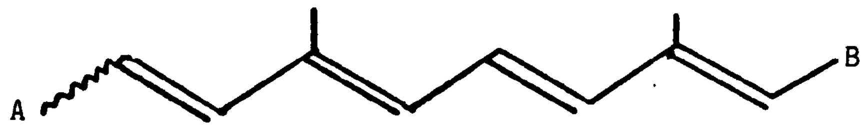

(III)

formule dans laquelle :

a) ou bien A est un groupement choisi parmi les groupements de formules :

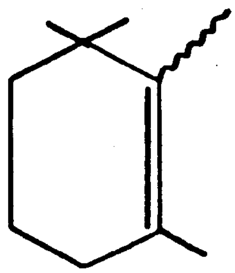

(IVa)

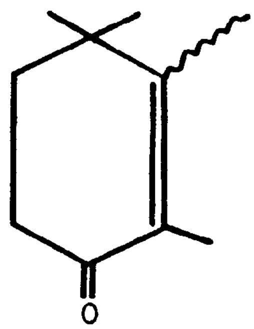

(V)

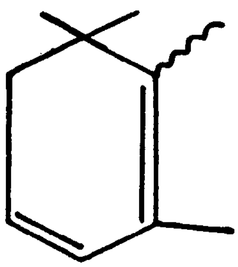

(IVb)

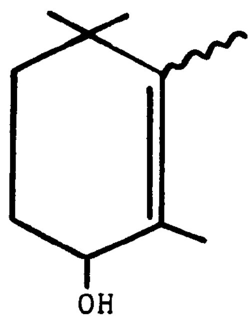

(VI)

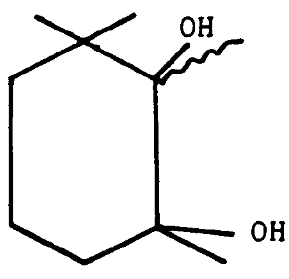

(VII)

B étant alors choisi :

aa) si A est un groupement de formule IVa :

- parmi les groupements suivants :

. -CHO ;

. $-CH_2OR_9$, dans lequel $R_9$ est l'hydrogène ou un radical alkyle en $C_1$-$C_4$ ;

.

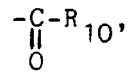

où $R_{10}$ est un radical alkyle linéaire ou ramifié en $C_1$-$C_{16}$ ;

. $-CH_2SR_{11}$, dans lequel $R_{11}$ est l'hydrogène ou un radical méthyle ;

.

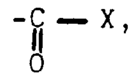

dans lequel X désigne :

(i) -OH ;

(ii) $-OR_{12}$ où $R_{12}$ est un radical alkyle en $C_1$-$C_{15}$, aryl(alkyle en $C_1$-$C_4$), substitué ou non sur le groupement aryle, arylcarboxy (alkyle en $C_1$-$C_4$), substitué ou non sur le groupement aryle, hydroxy(alkyle en $C_1$-$C_4$), amido(alkyle en $C_1$-$C_4$) ;

(iii) $NR_{13}R_{14}$, dans lequel $R_{13}$ ou $R_{14}$, identiques ou différents, désignent l'hydrogène, un radical alkyle en $C_1$-$C_6$, hydroxyalkyle en $C_1$-$C_4$, aryle substitué ou non, un hétérocycle substitué ou non ou dans lequel $R_{13}$ et $R_{14}$ forment, conjointement avec l'atome d'azote auquel ils sont reliés, un hétérocycle substitué ou non ;

(iv) le groupement $N_3$ ;

. $-CH_2NHR_{15}$, où $R_{15}$ désigne un radical benzoyle substitué ou non ;

ab) si A est un groupement de formule IVb, V, VI ou VII : parmi -COOH et les formes salifiées ou estérifiées correspondantes ;

b) ou bien A est choisi dans le groupe formé par les groupements aryle, substitués ou non, les hétérocycles, substitués ou non, les groupements arylhétérocycliques, substitués ou non sur l'hétérocycle, ou les groupements arylhomocycliques, substitués ou non sur le noyau aromatique,

B étant alors choisi parmi les groupements: -COOH, $-COOR_{16}$ où $R_{16}$ est un radical alkyle en $C_1$-$C_4$ ou un radical amide substitué par un alkyle en $C_1$-$C_4$, ainsi que leurs sels et leurs esters physiologiquement acceptables.

**11.** Composition selon la revendication 10, caractérisée par le fait que le dérivé rétinoïde est un dérivé de formule (III) pour lequel (alkyle en $C_1$-$C_4$) désigne méthyle, éthyle, n-butyle, t-butyle ; (alkyle en $C_1$-$C_{16}$) désigne éthyle, propyle, palmityle ; aryle désigne phényle ou benzyle, les substituants des groupements aryle étant (alkyle en $C_1$-$C_4$), (alcoxy en $C_1$-$C_{12}$), hydroxyle, halogène, nitro, les substituants alkyle ou alcoxy pouvant être eux-mêmes substitués par un groupement OH.

**12.** Composition selon la revendication 10, caractérisée par le fait que le dérivé rétinoïde est un dérivé de formule (III) comportant un groupement hétérocyclique choisi dans le groupe formé par les groupements dérivés de la phtalimide, de la succinimide et les hétérocycles de 4 à 6 chaînons comportant un ou plusieurs atomes d'oxygène et/ou un ou plusieurs atomes d'azote.

**13.** Composition selon la revendication 10, caractérisée par le fait que le dérivé rétinoïde est choisi dans le groupe formé par le rétinal ; le rétinol ; l'acétate, le proprionate et le palmitate de rétinyle ; l'acide rétinoïque, sous les formes tout-trans, 13-cis, 9-cis, 11-cis, 9, 13-dicis, 11,13-dicis ; les rétinoates de zinc correspondants ; le motrétinide ; l'étrétinate ; les rétinoates d'ammonium quaternaire de formule :

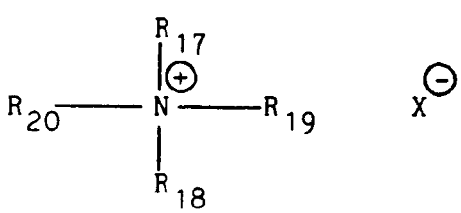

(VIII)

formule dans laquelle :

$X^{\ominus}$ désigne un radical rétinoate tout-trans ou 13-cis, et

i) ou bien $R_{17}$, $R_{18}$, $R_{19}$, identiques ou différents, sont un radical alkyle linéaire en $C_1$-$C_4$, substitué ou non par un ou plusieurs hydroxyle, $R_{20}$ étant un radical alkyle ou alcényle linéaire en $C_{12}$-$C_{18}$ ;
ii) ou bien $R_{19}$ est un groupement

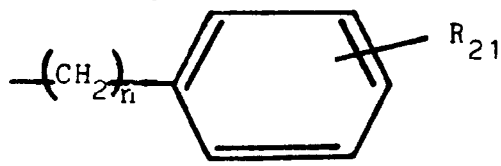

dans lequel :

n est égal à 0 ou 1 ;

$R_{21}$ représente un atome d'hydrogène ou d'halogène, un radical hydroxyle, alkyle ou hydroxyalkyle en $C_1$-$C_{18}$, ou acyle en $C_2$-$C_{18}$ ;

$R_{17}$, $R_{18}$ et $R_{19}$ ayant les significations indiquées sous i) ;

iii) ou bien $R_{17}$ et $R_{18}$ forment un hétérocycle aliphatique comportant au moins un atome d'oxygène, d'azote ou de soufre,
$R_{19}$ et $R_{20}$ ayant les significations indiquées sous i) et ii).

**14.** Composition selon la revendication 1, caractérisée par le fait qu'elle contient, comme dérivé de pyrimidine,le minoxidil et, comme dérivé rétinoïde, l'acide tout-trans rétinoïque.

**15.** Composition selon l'une des revendications 1 à 14, caractérisée par le fait que le dérivé rétinoïde de formule (III) dans laquelle B est -COOH, est présent dans des proportions comprises entre 0,005 % et 0,030 % en poids par rapport au poids total de la composition.

**16.** Composition selon l'une des revendications 1 à 15, caractérisée par le fait que la phase aqueuse D est gélifiée.

**17.** Composition selon la revendication 16, caractérisée par le fait que l'agent gélifiant est choisi dans le groupe formé par les hétérobiopolysaccharides, les dérivés de cellulose et les polymères acryliques réticulés ou non.

**18.** Composition selon l'une des revendications 16 ou 17, caractérisée par le fait que l'agent gélifiant est présent dans une proportion comprise entre 0,1 et 5 % en poids par rapport au poids total de la composition.

**19.** Composition selon l'une des revendications 1 à 18, caractérisée par le fait que la phase aqueuse D contient au moins un additif hydrosoluble physiologiquement acceptable.

**20.** Composition selon l'une des revendications 1 à 19, caractérisée par le fait que son pH est compris entre 4 et 9.

**21.** Utilisation d'une composition selon l'une des revendications 1 à 20 pour la fabrication d'un médicament destiné à induire et stimuler la croissance des cheveux et diminuer leur chute.

**22.** Procédé de traitement cosmétique des cheveux et du cuir chevelu, caractérisé par le fait qu'on leur applique par friction 1 à 5 g de la composition selon l'une des revendications 1 à 20 à une fréquence de une a deux applications par jour pendant 1 à 7 jours par semaine, pendant un temps de traitement compris entre 1 et 6 mois.

**Revendications pour les Etats contractants suivants : ES, GR**

**1.** Composition cosmétique pour application topique contenant, en association, au moins un dérivé rétinoïde et au moins un dérivé de pyrimidine, caractérisée par le fait qu'elle est constituée d'une dispersion de vésicules d'au moins un lipide amphiphile ionique et/ou non-ionique délimitées par un ou plusieurs feuillets lipidiques dans une phase aqueuse de dispersion physiologiquement acceptable D, le(s) dérivé(s) rétinoïde(s) se trouvant dans les feuillets lipidiques des vésicules, alors que le(s) dérivé(s) de pyrimidine est (ou sont) sous forme de solution ou, partiellement ou totalement, sous forme de suspension dans la phase

aqueuse D.

2. Composition selon la revendication 1, caractérisée par le fait que le(s) lipide(s) amphiphile(s) constitutif(s) des feuillets des vésicules est (ou sont) un (ou des) lipide(s) ionique(s) pris dans le groupe formé par les phospholipides naturels ou synthétiques.

3. Composition selon la revendication 1, caractérisée par le fait que le(s) lipide(s) amphiphile(s) constitutif(s) des feuillets des vésicules est (ou sont) un (ou des) lipide(s) non-ionique(s) pris dans le groupe formé par les composés de formule (I) :

$$RO-[C_3H_5(OH)O]_{\bar{n}}-OH \qquad (I)$$

- où $-C_3H_5(OH)O$ est représenté par les structures suivantes prises en mélange ou séparément: $-CH_2CHOH\ CH_2O-$ ;

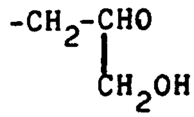 ; 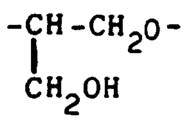

- où $\bar{n}$ est une valeur statistique moyenne comprise entre 2 et 6 ;
- où R est :
  a) soit une chaîne aliphatique $R_1$ ou un reste $R_2CO$, $R_1$ étant un radical aliphatique, linéaire ou ramifié, en $C_{12}$-$C_{18}$ et $R_2$ étant un radical aliphatique, linéaire ou ramifié, en $C_{11}$-$C_{17}$ ;
  b) soit

$$R_3-[OC_2H_3(R_4)]-$$

  où $-OC_2H_3(R_4)-$ est représenté par les structures suivantes prises en mélange ou séparément :

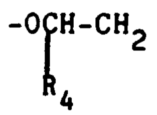 et 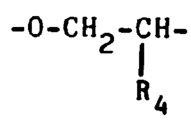

  $R_3$ étant un radical $R_1$ ou $R_2CO$, $R_4$ étant un radical $R_1$, et $R_1$ et $R_2$ ayant les significations définies ci-dessus,

et les phytostérols oxyéthylénés.

4. Composition selon l'une des revendications 1 à 3, caractérisée par le fait que le(s) lipide(s) amphiphile(s) des feuillets est (ou sont) associé(s) à au moins un additif modifiant la perméabilité ou la charge superficielle des vésicules.

5. Composition selon l'une des revendications 1 à 4, caractérisée par le fait que le poids total de lipide(s) formant les vésicules, y compris éventuellement l'(ou les) additif(s) associé(s), représente entre 0,2 et 20 % du poids total de la composition.

6. Composition selon l'une des revendications 1 à 5, caractérisée par le fait qu'elle contient au moins un dérivé de pyrimidine de formule :

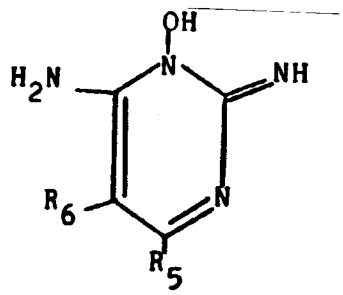

(II)

formule dans laquelle :

- $R_5$ représente un groupement

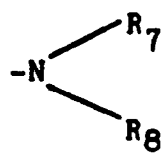

  dans lequel $R_7$ et $R_5$ sont choisis parmi l'hydrogène, un groupement alkyle en $C_1$-$C_4$, alcényle en $C_2$-$C_5$, (alkyle en $C_1$-$C_4$)aryle, et cycloalkyle en $C_4$-$C_7$ ou forment un hétérocycle avec l'atome d'azote auquel ils sont liés choisi parmi les groupements aziridinyle, azétidinyle, pyrrolidinyle, pipéridinyle, hexahydroazépinyle, heptaméthylèneimine, octaméthylèneimine, morpholine ou (alkyl en $C_1$-$C_4$)-4-pipérazinyle, les groupements hétérocycliques pouvant entre substitués sur les atomes de carbone par un à trois groupements alkyle en $C_1$-$C_4$,hydroxy ou alcoxy en $C_1$-$C_4$;
- $R_5$ représente l'hydrogène, un groupement alkyle en $C_1$-$C_4$, alcényle en $C_2$-$C_5$ cycloalkyle en $C_4$-$C_7$, aryle, (alkyl en $C_1$-$C_4$)aryle, aryl(alkyle en $C_1$-$C_4$), (alkyl en $C_1$-$C_4$)aryl(alkyle en $C_1$-$C_4$), alcoxy(alkyle en $C_1$-$C_4$) (alcoxy en $C_1$-$C_4$)aryl (alkyle en $C_1$-$C_4$) ou haloaryl(alkyle en $C_1$-$C_4$) et/ou au moins un sel d'addition d'acides physiologiquement acceptables.

**7.** Composition selon la revendication 6, caractérisée par le fait qu'elle contient l'amino-6 dihydro-1,2 hydroxy-1 imino-2 pipéridino-4 pyrimidine et/ou au moins un de ses sels d'addition physiologiquement acceptables.

**8.** Composition selon l'une des revendications 1 à 7, caractérisée par le fait qu'elle contient au moins un dérivé de pyrimidine, qui est, au moins partiellement, sous forme de suspension, dans la phase aqueuse D, de particules ayant une granulométrie inferieure à 80 µm.

**9.** Composition selon l'une des revendications 1 à 8, caractérisée par le fait que le(s) dérivé(s) de pyrimidine est (ou sont) présent(s) dans la composition dans des proportions comprises entre 0,05 et 6 % en poids par rapport au poids total de la composition.

**10.** Composition selon l'une des revendications 1 à 9, caractérisée par le fait que le dérivé rétinoïde est un dérivé de formule (III) :

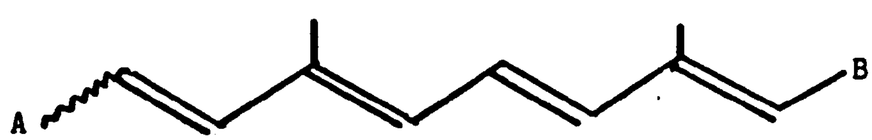

(III)

formule dans laquelle :

a) ou bien A est un groupement choisi parmi les groupements de formules :

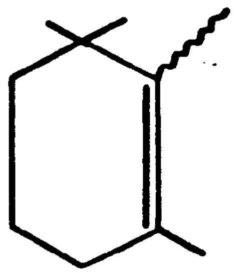

(IVa)

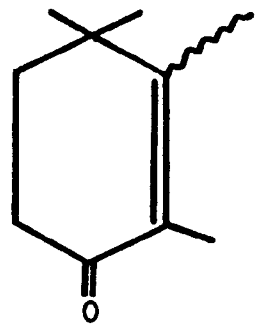

(V)

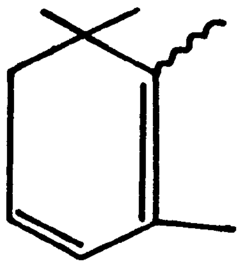

(IVb)

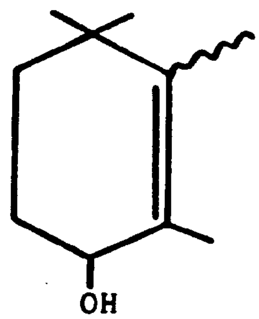

(VI)

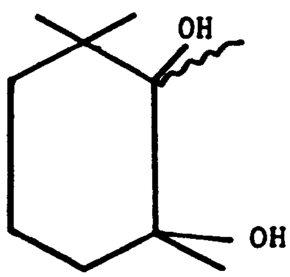

(VII)

B étant alors choisi :

aa) si A est un groupement de formule IVa :

- parmi les groupements suivants :

. -CHO ;

. $-CH_2OR_9$, dans lequel $R_9$ est l'hydrogène ou un radical alkyle en $C_1$-$C_4$ ;

.

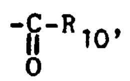

où $R_{10}$ est un radical alkyle linéaire ou ramifié en $C_1$-$C_{16}$ ;

. $-CH_2SR_{11}$, dans lequel $R_{11}$ est l'hydrogène ou un radical méthyle ;

.

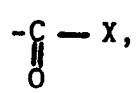

dans lequel X désigne :

(i) -OH ;

(ii) $-OR_{12}$ où $R_{12}$ est un radical alkyle en $C_1$-$C_{15}$, aryl(alkyle en $C_1$-$C_4$), substitué ou non sur

le groupement aryle, arylcarboxy (alkyle en $C_1$-$C_4$), substitué ou non sur le groupement aryle, hydroxy(alkyle en $C_1$-$C_4$), amido(alkyle en $C_1$-$C_4$)

(iii) $NR_{13}R_{14}$, dans lequel $R_{13}$ ou $R_{14}$, identiques ou différents désignent l'hydrogène, un radical alkyle en $C_1$- $C_6$, hydroxyalkyle en $C_1$-$C_4$, aryle substitué ou non, un hétérocycle substitué ou non ou dans lequel $R_{13}$ et $R_{14}$ forment, conjointement avec l'atome d'azote auquel ils sont reliés, un hétérocycle substitué ou non ;

(iv) le groupement $N_3$;

. $-CH_2NHR_{15}$, où $R_{15}$ désigne un radical benzoyle substitué ou non ;

ab)si A est un groupement de formule IVb, V, VI ou VII : parmi -COOH et les formes salifiées ou estérifiées correspondantes

b) ou bien A est choisi dans le groupe formé par les groupements aryle, substitués ou non, les hétérocycles, substitués ou non, les groupements arylhétérocycliques, substitués ou non sur l'hétérocycle, ou les groupements arylhomocycliques, substitués ou non sur le noyau aromatique,

B étant alors choisi parmi les groupements: -COOH, $-COOR_{16}$ où $R_{16}$ est un radical alkyle en $C_1$-$C_4$ ou un radical amide substitué par un alkyle en $C_1$-$C_4$, ainsi que leurs sels et leurs esters physiologiquement acceptables.

**11.** Composition selon la revendication 10, caractérisée par le fait que le dérivé rétinoïde est un dérivé de formule (III) pour lequel (alkyle en $C_1$-$C_4$) désigne méthyle, éthyle, n-butyle, t-butyle ; (alkyle en $C_1$-$C_{16}$) désigne éthyle, propyle, palmityle ; aryle désigne phényle ou benzyle, les substituants des groupements aryle étant (alkyle en $C_1$-$C_4$) , (alcoxy en $C_1$- $C_{12}$), hydroxyle, halogène, nitro, les substituants alkyle ou alcoxy pouvant entre eux-mêmes substitués par un groupement OH.

**12.** Composition selon la revendication 10, caractérisée par le fait que le dérivé rétinoïde est un derivé de formule (III) comportant un groupement hétérocyclique choisi dans le groupe formé par les groupements dérivés de la phtalimide, de la succinimide et les hétérocycles de 4 à 6 chaînons comportant un ou plusieurs atomes d'oxygène et/ou un ou plusieurs atomes d'azote.

**13.** Composition selon la revendication 10, caractérisée par le fait que le dérivé rétinoïde est choisi dans le groupe formé par le rétinal ; le rétinol ; l'acétate, le proprionate et le palmitate de rétinyle ; l'acide rétinoïque, sous les formes tout-trans, 13-cis, 9-cis, 11-cis, 9,13-dicis, 11,13-dicis ; les rétinoates de zinc correspondants ; le motrétinide ; l'étrétinate ; les rétinoates d'ammonium quaternaire de formule :

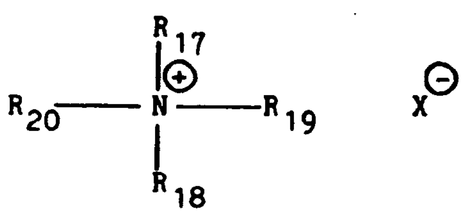

(VIII)

formule dans laquelle :

$X^{\ominus}$ désigne un radical rétinoate tout-trans ou 13-cis, et

i) ou bien $R_{17}$, $R_{18}$, $R_{19}$, identiques ou différents, sont un radical alkyle linéaire en $C_1$-$C_4$, substitué ou non par un ou plusieurs hydroxyle, $R_{20}$ étant un radical alkyle ou alcényle linéaire en $C_{12}$-$C_{18}$ ;

ii) ou bien $R_{19}$ est un groupement

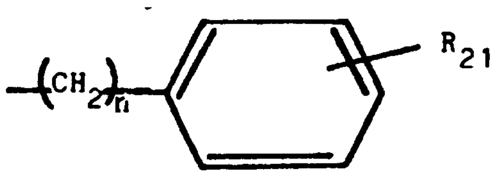

dans lequel :

n est égal à 0 ou 1 ;

$R_{21}$ représente un atome d'hydrogène ou d'halogène, un radical hydroxyle, alkyle ou hy-

droxyalkyle en $C_1$-$C_{18}$, ou acyle en $C_2$-$C_{18}$ ;

$R_{17}$, $R_{18}$ et $R_{19}$ ayant les significations indiquées sous i) ;

iii) ou bien $R_{17}$ et $R_{18}$ forment un hétérocycle aliphatique comportant au moins un atome d'oxygène, d'azote ou de soufre,

$R_{19}$ et $R_{20}$ ayant les significations indiquées sous i) et ii).

**14.** Composition selon la revendication 1, caractérisée par le fait qu'elle contient, comme dérivé de pyrimidine,le minoxidil et,comme dérivé rétinoïde, l'acide tout-trans rétinoïque.

**15.** Composition selon l'une des revendications 1 à 14, caractérisée par le fait que le dérivé rétinoïde de formule (III) dans laquelle B est -COOH, est présent dans des proportions comprises entre 0,005 % et 0,030 % en poids par rapport au poids total de la composition.

**16.** Composition selon l'une des revendications 1 à 15, caractérisée par le fait que la phase aqueuse D est gélifiée.

**17.** Composition selon la revendication 16, caractérisée par le fait que l'agent gélifiant est choisi dans le groupe formé par les hétérobiopolysaccharides, les dérivés de cellulose et les polymeres acryliques réticulés ou non.

**18.** Composition selon l'une des revendications 16 ou 17, caractérisée par le fait que l'agent gélifiant est présent dans une proportion comprise entre 0,1 et 5 % en poids par rapport au poids total de la composition.

**19.** Composition selon l'une des revendications 1 à 18, caractérisée par le fait que la phase aqueuse D contient au moins un additif hydrosoluble physiologiquement acceptable.

**20.** Composition selon l'une des revendications 1 à 19, caractérisée par le fait que son pH est compris entre 4 et 9.

**21.** Procédé de traitement cosmétique des cheveux et du cuir chevelu, caractérisé par le fait qu'on leur applique par friction 1 à 5 g de la composition selon l'une des revendications 1 à 20 à une fréquence de une à deux applications par jour pendant 1 à 7 jours par semaine, pendant un temps de traitement compris entre 1 et 6 mois.

## Claims

### Claims for the following Contracting States : AT, BE, CH, DE, DK, FR, GB, IT, LI, NL, SE

**1.** Cosmetic or pharmaceutical composition for topical application containing, in combination, at least one retinoid derivative and at least one pyrimidine derivative, characterized in that it consists of a dispersion of vesicles of at least one ionic and/or nonionic amphiphilic lipid, the vesicles being bounded by one or more lipid lamella(e) in a physiologically acceptable aqueous dispersion phase D, the retinoid derivative(s) being in the lipid lamellae of the vesicles whereas the pyrimidine derivative(s) is (or are) in the form of a solution or, partially or totally, in the form of a suspension in the aqueous phase D.

**2.** Composition according to Claim 1, characterized in that the amphiphilic lipid(s) forming the lamellae of the vesicles is (or are) one (or more) ionic lipid(s) selected from the group composed of natural or synthetic phospholipids.

**3.** Composition according to Claim 1, characterized in that the amphiphilic lipid(s) forming the lamellae of the vesicles is (or are) one (or more) nonionic lipid(s) selected from the group composed of the compounds of formula (I):

$$RO-\left[C_3H_5(CH)\ C\right]_{\bar{n}}-OH \qquad (I)$$

- where $-C_3H_5(OH)$ O- is represented by the following structures, taken mixed or separately: $-CH_2CHOHCH_2O-$;

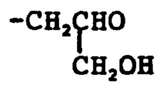 ; 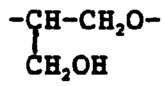

- where $\bar{n}$ is an average statistical value between 2 and 6;
- where R is:

a) either an aliphatic chain $R_1$ or a residue $R_2CO$, $R_1$ being a linear or branched $C_{12}$-$C_{18}$ aliphatic radical and $R_2$ being a linear or branched $C_{11}$-$C_{17}$ aliphatic radical;

b) or $R_3\{O - C_2H_3(R_4)\}$ where $-OCH_2H_3(R_4)-$ is represented by the following structures, taken mixed or separately:

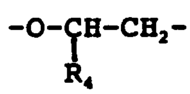 and 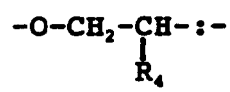

$R_3$ being a radical $R_1$ or $R_2CO$, $R_4$ being a radical $R_1$, and $R_1$ and $R_2$ having the meanings defined above; and oxyethylenated phytosterols.

4. Composition according to one of Claims 1 to 3, characterized in that the amphiphilic lipid(s) of the lamellae is (or are) combined with at least one additive modifying the permeability or the surface charge of the vesicles.

5. Composition according to one of Claims 1 to 4, characterized in that the total weight of lipid(s) forming the vesicles, including, where appropriate, the additive(s) combined therewith, represents between 0.2 and 20 % of the toal weight of the composition.

6. Composition according to one of Claims 1 to 5, characterized in that it contains at least one pyrimidine derivative of the formula:

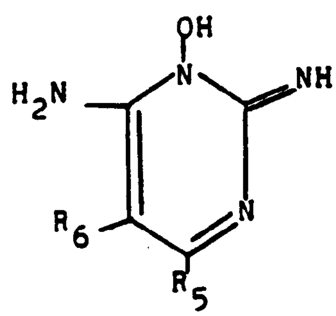

(II)

in which formula:

- $R_5$ represents a group

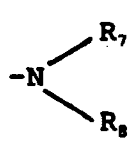 ,

in which $R_7$ and $R_5$ are selected from hydrogen and a $C_1$-$C_4$ alkyl, $C_2$-$C_5$ alkenyl, ($C_1$-$C_4$ alkyl)aryl and $C_4$-$C_7$ cycloalkyl group, or form a heterocycle with the nitrogen atom to which they are linked, selected from aziridinyl, azetidinyl, pyrrolidinyl, piperidyl, hexahydroazepinyl, heptamethylenimine, octamethylenimine, morpholine or 4-($C_1$-$C_4$ alkyl) piperazinyl groups, it being possible for the heterocyclic groups to be substituted on the carbon atoms with one to three $C_1$-$C_4$ alkyl, hydroxy or $C_1$-$C_4$ alkoxy groups; and

- $R_6$ represents hydrogen or a $C_1$-$C_4$ alkyl, $C_2$-$C_5$ alkenyl, $C_4$-$C_7$ cycloalkyl, aryl, ($C_1$-$C_4$ alkyl)aryl, aryl ($C_1$-$C_4$ alkyl), ($C_1$-$C_4$ alkyl)aryl ($C_1$-$C_4$ alkyl), alkoxy ($C_1$-$C_4$ alkyl), ($C_1$-$C_4$ alkoxy)aryl ($C_1$-$C_4$ alkyl) or haloaryl($C_1$-$C_4$ alkyl) group; and/or at least one addition salt with physiologically acceptable acids.

**7.** Composition according to Claim 6, characterized in that it contains 6-amino-1,2-dihydro-1-hydroxy-2-imino-4-piperidinopyrimidine and/or at least one of its physiologically acceptable addition salts.

**8.** Composition according to one of Claims 1 to 7, characterized in that it contains at least one pyrimidine derivative which is at least partially in the form of a suspension, in the aqueous phase D, of particles having a particle size of less than 80 μm.

**9.** Composition according to one of Claims 1 to 8, characterized in that the pyrimidine derivative(s) is (or are) present in the composition in proportions of between 0.05 and 6 % by weight relative to the total weight of the composition.

**10.** Composition according to one of Claims 1 to 9, characterized in that the retinoid derivative is a derivative of formula (III):

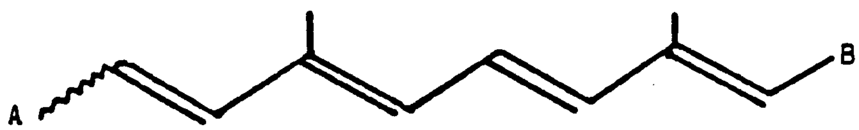

(III)

in which formula:

a) either A is a group selected from the groups of formulae:

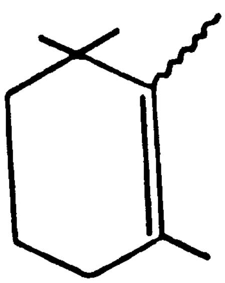

(IVa)

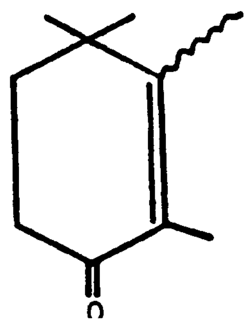

(V)

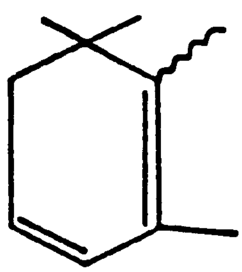

(IVb)

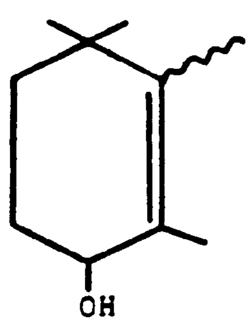

(VI)

(VII)

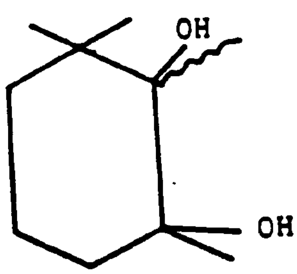

B then being selected:

aa) if A is a group of formula IVa:

- from the following groups:

● -CHO;

● $-CH_2OR_9$, in which $R_9$ is hydrogen or a $C_1$-$C_4$ alkyl radical;

●

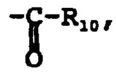

where $R_{10}$ is a linear or branched $C_1$-$C_{16}$ alkyl radical;

● $-CH_2SR_{11}$, in which $R_{11}$ is hydrogen or a methyl radical;

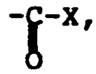

in which X denotes:

(i) -OH;

(ii) $-OR_{12}$, where $R_{12}$ is a $C_1$-$C_{15}$ alkyl radical, an aryl($C_1$-$C_4$ alkyl) radical substituted or unsubstituted on the aryl group, an arylcarboxy($C_1$-$C_4$ alkyl) radical substituted or unsubstituted on the aryl group, or a hydroxy($C_1$-$C_4$ alkyl) or amido($C_1$-$C_4$ alkyl) radical;

(iii) $-NR_{13}R_{14}$, in which $R_{13}$ or $R_{14}$, which may be identical or different, denote hydrogen, a $C_1$-$C_6$ alkyl, $C_1$-$C_4$ hydroxyalkyl or substituted or unsubstituted aryl radical or a substituted or unsubstituted heterocycle, or in which $R_{13}$ and $R_{14}$, together with the nitrogen atom to which they are attached, form a substituted or unsubstituted heterocycle;

(iv) an $N_3$ group;

● $-CH_2NHR_{15}$, where $R_{15}$ denotes a substituted or unsubstituted benzoyl radical;

ab) if A is a group of formula IVb, V, VI or VII: from -COOH and the corresponding salified or esterified forms;

b) or A is selected from the group composed of substituted or unsubstituted aryl groups, substituted or unsubstituted heterocycles, arylheterocyclic groups substituted or unsubstituted on the heterocycle or arylhomocyclic groups substituted or unsubstituted on the aromatic ring,

B then being selected from the groups -COOH and $-COOR_{16}$, where $R_{16}$ is a $C_1$-$C_4$ alkyl radical or an amide radical substituted with a $C_1$-$C_4$ alkyl; as well as their physio-logically acceptable salts and esters.

**11.** Composition according to Claim 10, characterized in that the retinoid derivative is a derivative of formula (III) for which ($C_1$-$C_4$ alkyl) denotes methyl, ethyl, n-butyl, t-butyl; ($C_1$-$C_{16}$ alkyl) denotes ethyl, propyl, palmityl; aryl denotes phenyl or benzyl, the substituents on the aryl groups being ($C_1$-$C_4$ alkyl), ($C_1$-$C_{12}$ alkoxy) , hydroxyl, halogen or nitro, it being possible for the alkoxy or alkyl substituents themselves to be substituted with an OH group.

**12.** Composition according to Claim 10, characterized in that the retinoid derivative is a derivative of formula (III) containing a heterocyclic group selected from the group composed of groups derived from phthalimide and from succinimide and 4- to 6-membered heterocycles containing one or more oxygen atoms and/or one or more nitrogen atoms.

**13.** Composition according to Claim 10, characterized in that the retinoid derivative is selected from the group composed of retinal; retinol; retinyl acetate, propionate and palmitate; retinoic acid in all-trans, 13-cis, 9-cis, 11-cis, 9,13-dicis, 11,13-dicis forms; the corresponding zinc retinoates; motretinide; etretinate; the quaternary ammonium retinoates of formula:

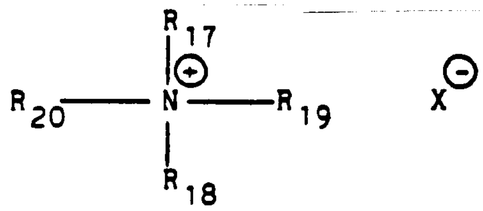

(VIII)

in which formula:
$X^{\ominus}$ denotes an all-trans- or 13-cis-retinoate radical, and
i) either $R_{17}$, $R_{18}$ and $R_{19}$, which may be identical or different, are a linear $C_1$-$C_4$ alkyl radical substituted or unsubstituted with one or more hydroxyls, $R_{20}$ being a linear $C_{12}$-$C_{18}$, alkyl or alkenyl radical;
ii) or $R_{19}$ is a group

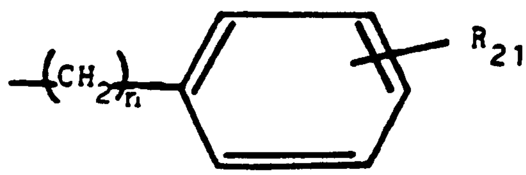

in which: n is equal to 0 or 1,
$R_{21}$ represents a hydrogen or halogen atom or a hydroxyl, $C_1$-$C_{18}$, alkyl or hydroxyalkyl or $C_2$-$C_{18}$ acyl radical;
$R_{17}$, $R_{18}$ and $R_{19}$ having the meanings stated under
iii) or $R_{17}$ and $R_{18}$ form an aliphatic heterocycle containing at least one oxygen, nitrogen or sulphur atom, $R_{19}$ and $R_{20}$ having the meanings stated under i) and ii).

**14.** Composition according to Claim 1, characterized in that it contains minoxidil as a pyrimidine derivative, and all-trans-retinoic acid as a retinoid derivative.

**15.** Composition according to one of Claims 1 to 14, characterized in that the retinoid derivative of formula (III) in which B is -COOH is present in proportions of between 0.005 % and 0.030 % by weight relative to the total weight of the composition.

**16.** Composition according to one of Claims 1 to 15, characterized in that the aqueous phase D is gelled.

**17.** Composition according to Claim 16, characterized in that the gelling agent is selected from the group composed of heterobiopolysaccharides, cellulose derivatives and acrylic polymers, crosslinked or otherwise.

**18.** Composition according to one of Claims 16 and 17, characterized in that the gelling agent is present in a proportion of between 0.1 and 5 % by weight relative to the total weight of the composition.

**19.** Composition according to one of Claims 1 to 18, characterized in that the aqueous phase D contains at least one physiologically acceptable, water-soluble additive.

**20.** Composition according to one of Claims 1 to 19, characterized in that its pH is between 4 and 9.

**21.** Use of a composition according to one of Claims 1 to 20 for the manufacture of a medicinal product intended for inducing and stimulating hair growth and decreasing its loss.

**22.** Process for cosmetic treatment of the hair and scalp, characterized in that 1 to 5 g of the composition according to one of Claims 1 to 20 is applied thereto by massage at a frequency of one to two applications per day for 1 to 7 days per week, for a treatment period of between 1 and 6 months.

**Claims for the following Contracting States : ES, GR**

**1.** Cosmetic composition for topical application containing, in combination, at least one retinoid derivative and at least one pyrimidine derivative, characterized in that it consists of a dispersion of vesicles of at least

one ionic and/or nonionic amphiphilic lipid, the vesicles being bounded by one or more lipid lamella(e) in a physiologically acceptable aqueous dispersion phase D, the retinoid derivative(s) being in the lipid lamellae of the vesicle whereas the pyrimidine derivative(s) is (or are) in the form of a solution or, partially or totally, in the form of a suspension in the aqueous phase D.

2. Composition according to Claim 1, characterized in that the amphiphilic lipid(s) forming the lamellae of the vesicles is (or are) one (or more) ionic lipid(s) selected from the group composed of natural or synthetic phospholipids.

3. Composition according to Claim 1, characterized in that the amphiphilic lipid(a) forming the lamellae of the vesicles is (or are) one (or more) nonionic lipid(s) selected from the group composed of the compounds of formula (I):

$$RO-[C_3H_5(OH)O]_{\bar{n}}-OH \qquad (I)$$

- where $-C_3H_5(OH)O-$ is represented by the following structures, taken mixed or separately: $-CH_2CHOHCH_2O-$;

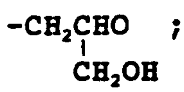 ; 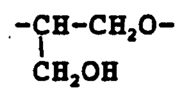

- where $\bar{n}$ is an average statistical value between 2 and 6;
- where R is:

a) either an aliphatic chain $R_1$ or a residue $R_2CO$, $R_1$ being a linear or branched $C_{12}$-$C_{18}$ aliphatic radical and $R_2$ being a linear or branched $C_{11}$-$C_{17}$ aliphatic radical;

b) or $R_3[O - C_2H_3(R_4)]$ where $-OCH_2H_3(R_4)-$ is represented by the following structures, taken mixed or separately:

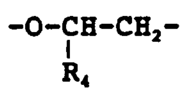 and 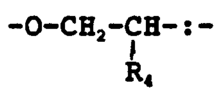

$R_3$ being a radical $R_1$ or $R_2CO$, $R_4$ being a radical $R_1$, and $R_1$ and $R_2$ having the meanings defined above; and oxyethylenated phytosterols.

4. Composition according to one of Claims 1 to 3, characterized in that the amphiphilic lipid(s) of the lamellae is (or are) combined with at least one additive modifying the permeability or the surface charge of the vesicles.

5. Composition according to one of Claims 1 to 4, characterized in that the total weight of lipid(s) forming the vesicles, including, where appropriate, the additive(s) combined therewith, represents between 0.2 and 20 % of the toal weight of the composition.

6. Composition according to one of Claims 1 to 5, characterized in that it contains at least one pyrimidine derivative of the formula:

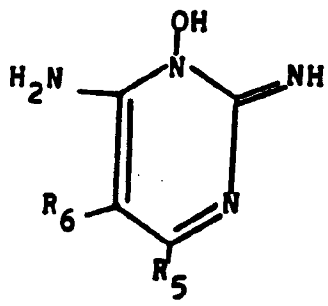

(II)

in which formula:

- $R_5$ represents a group

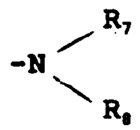

,

in which $R_7$, and $R_5$ are selected from hydrogen and a $C_1$-$C_4$ alkyl, $C_2$-$C_5$ alkenyl, ($C_1$-$C_4$ alkyl) aryl and $C_4$-$C_7$ cycloalkyl group, or form a heterocycle with the nitrogen atom to which they are linked, selected from aziridinyl, azetidinyl, pyrrolidinyl, piperidyl, hexahydroazepinyl, heptamethylenimine, octamethylenimine, morpholine or 4-($C_1$-$C_4$ alkyl) piperazinyl groups, it being possible for the heterocyclic groups to be substituted on the carbon atoms with one to three $C_1$-$C_4$ alkyl, hydroxy or $C_1$-$C_4$ alkoxy groups; and

- $R_5$ represents hydrogen or a $C_1$-$C_4$ alkyl, $C_2$-$C_5$ alkenyl, $C_4$-$C_7$ cycloalkyl, aryl, ($C_1$-$C_4$ alkyl)aryl, aryl($C_1$-$C_4$ alkyl), ($C_1$-$C_4$ alkyl)aryl($C_1$-$C_4$ alkyl), alkoxy($C_1$-$C_4$ alkyl), ($C_1$-$C_4$ alkoxy)aryl($C_1$-$C_4$ alkyl) or haloaryl($C_1$-$C_4$ alkyl) group; and/or at least one addition salt with physiologically acceptable acids.

7. Composition according to Claim 6, characterized in that it contains 6-amino-1,2-dihydro-1-hydroxy-2-imino-4-piperidinopyrimidine and/or at least one of its physiologically acceptabla addition salts.

8. Composition according to one of Claims 1 to 7, characterized in that it contains at least one pyrimidine derivative which is at least partially in the form of a suspension, in the aqueous phase D, of particles having a particle size of less than 80 µm.

9. Composition according to one of Claims 1 to 8, characterized in that the pyrimidine derivative(s) is (or are) present in the composition in proportions of between 0.05 and 6 % by weight relative to the total weight of the composition.

10. Composition according to one of Claims 1 to 9, characterized in that the retinoid derivative is a derivative of formula (III):

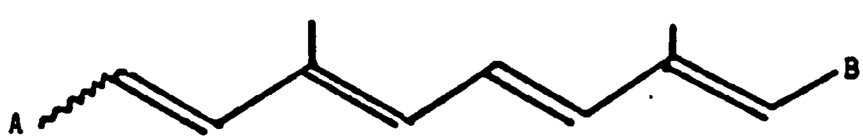

(III)

in which formula:

a) either A is a group selected from the groups of formulae:

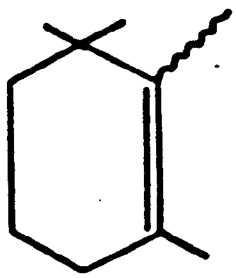

(IVa)

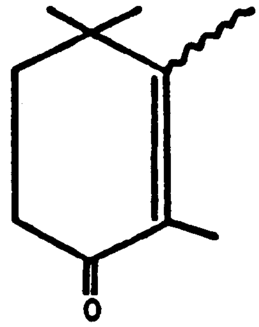

(V)

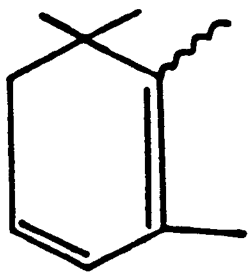

(IVb)

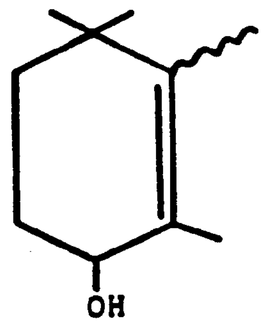

(VI)

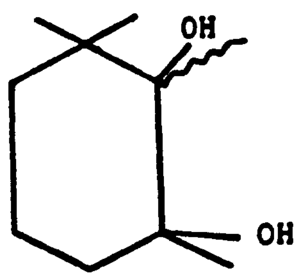

(VII)

B then being selected:

aa) if A is a group of formula IVa:

- from the following groups:

- $-CHO$;
- $-CH_2OR_9$, in which $R_9$ is hydrogen or a $C_1$-$C_4$ alkyl radical;
- 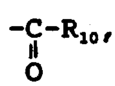

  where $R_{10}$ is a linear or branched $C_1$-$C_{16}$ alkyl radical;
- $-CH_2SR_{11}$, in which $R_{11}$ is hydrogen or a methyl radical;
- 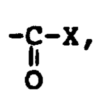

  in which X denotes:

  (i) -OH;

  (ii) $-OR_{12}$, where $R_{12}$ is a $C_1$-$C_{15}$ alkyl radical, an aryl($C_1$-$C_4$ alkyl) radical substituted or unsubstituted on the aryl group, an arylcarboxy($C_1$-$C_4$ alkyl) radical substituted or unsubstituted

on the aryl group, or a hydroxy($C_1$-$C_4$ alkyl) or amido($C_1$-$C_4$ alkyl) radical;

(iii) -$NR_{13}R_{14}$, in which $R_{13}$ or $R_{14}$, which may be identical or different, denote hydrogen, a $C_1$-$C_6$ alkyl, $C_1$-$C_4$ hydroxyalkyl or substituted or unsubstituted aryl radical or a substituted or unsubstituted heterocycle, or in which $R_{13}$ and $R_{14}$, together with the nitrogen atom to which they are attached, form a substituted or unsubstituted heterocycle;

(iv) an $N_3$ group;

● -$CH_2NHR_{15}$, where $R_{15}$ denotes a substituted or unsubstituted benzoyl radical;

ab) if A is a group of formula IVb, V, VI or VII: from -COOH and the corresponding salified or esterified forms;

b) or A is selected from the group composed of substituted or unsubstituted aryl groups, substituted or unsubstituted heterocycles, arylheterocyclic groups substituted or unsubstituted on the heterocycle or arylhomocyclic groups substituted or unsubstituted on the aromatic ring,

B then being selected from the groups -COOH and -$COOR_{16}$, where $R_{16}$ is a $C_1$-$C_4$ alkyl radical or an amide radical substituted with a $C_1$-$C_4$ alkyl; as well as their physiologically acceptable salts and esters.

**11.** Composition according to Claim 10, characterized in that the retinoid derivative is a derivative of formula (III) for which ($C_1$-$C_4$ alkyl) denotes methyl, ethyl, n-butyl, t-butyl; ($C_1$-$C_{16}$ alkyl) denotes ethyl, propyl, palmityl; aryl denotes phenyl or benzyl, the substituents on the aryl groups being ($C_1$-$C_4$ alkyl), ($C_1$-$C_{12}$ alkoxy), hydroxyl, halogen or nitro, it being possible for the alkoxy or alkyl substituents themselves to be substituted with an OH group.

**12.** Composition according to Claim 10, characterized in that the retinoid derivative is a derivative of formula (III) containing a heterocyclic group selected from the group composed of groups derived from phthalimide and from succinimide and 4- to 6-membered heterocycles containing one or more oxygen atoms and/or one or more nitrogen atoms.

**13.** Composition according to Claim 10, characterized in that the retinoid derivative is selected from the group composed of retinal; retinol; retinyl acetate, propionate and palmitate; retinoic acid in all-trans, 13-cis, 9-cis, 11-cis, 9,13-dicis, 11,13-dicis forms; the corresponding zinc retinoates; motretinide; etretinate; the quaternary ammonium retinoates of formula:

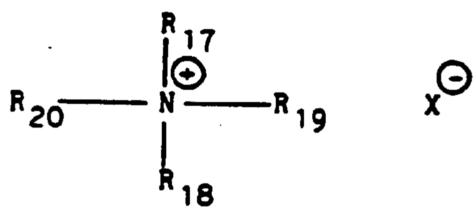

(VIII)

in which formula:

$X^{\ominus}$ denotes an all-trans- or 13-cis-retinoate radical, and

i) either $R_{17}$, $R_{18}$ and $R_{19}$, which may be identical or different, are a linear $C_1$-$C_4$ alkyl radical substituted or unsubstituted with one or more hydroxyls, $R_{20}$ being a linear $C_{12}$-$C_{18}$ alkyl or alkenyl radical;

ii) or $R_{19}$ is a group

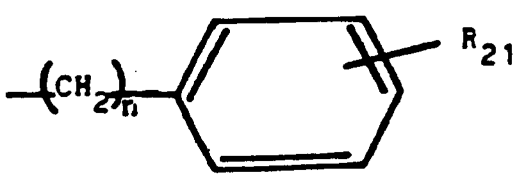

in which: n is equal to 0 or 1,

$R_{21}$ represents a hydrogen or halogen atom or a hydroxyl, $C_1$-$C_{18}$ alkyl or hydroxyalkyl or $C_2$-$C_{18}$ acyl radical;

$R_{17}$, $R_{18}$ and $R_{19}$ having the meanings stated under i);

iii) or $R_{17}$ and $R_{18}$ form an aliphatic heterocycle containing at least one oxygen, nitrogen or sulphur atom, $R_{19}$ and $R_{20}$ having the meanings stated under i) and ii).

**14.** Composition according to Claim 1, characterized in that it contains minoxidil as a pyrimidine derivative, and all-trans-retinoic acid as a retinoid derivative.

**15.** Composition according to one of Claims 1 to 14, characterized in that the retinoid derivative of formula (III) in which B is -COOH is present in proportions of between 0.005 % and 0.030 % by weight relative to the total weight of the composition.

**16.** Composition according to one of Claims 1 to 15, characterized in that the aqueous phase D is gelled.

**17.** Composition according to Cla im 16, characterized in that the gelling agent is selected from the group composed of heterobiopolysaccharides, cellulose derivatives and acrylic polymers, crosslinked or otherwise.

**18.** Composition according o one of Claims 16 and 17, characterized in that the gelling agent is present in a proportion of between 0.1 and 5 % by weight relative to the total weight of the composition.

**19.** Composition according to one of Claims 1 to 18, characterized in that the aqueous phase D contains at least one physiologically acceptable, water-soluble additive.

**20.** Composition according to one of Claims 1 to 19, characterized in that its pH is between 4 and 9.

**21.** Process for cosmetic treatment of the hair and scalp, characterized in that 1 to 5 g of the composition according to one of Claims 1 to 20 is applied thereto by massage at a frequency of one to two applications per day for 1 to 7 days per week, for a treatment period of between 1 and 6 months.

**Patentansprüche**

**Patentansprüche für folgende Vertragsstaaten : AT, BE, CH, DE, FR, GB, IT, LI, LU, NL, SE**

**1.** Kosmetisches oder pharmazeutisches Mittel zur topischen Anwendung, enthaltend in Kombination wenigstens ein Retinoidderivat und wenigstens ein Pyrimidinderivat, dadurch gekennzeichnet, daß es aus einer Dispersion von Vesikeln aus wenigstens einem ionischen und/oder nichtionischen amphiphilen Lipid, welche durch ein oder mehrere Lipidplättchen begrenzt sind, in einer wäßrigen physiologisch akzeptablen Dispersionsphase D gebildet ist, wobei sich das Retinoidderivat (die Retinoidderivate) in den Lipidplättchen der Vesikel befindet (befinden), während das Pyrimidinderivat (die Pyrimidinderivate) in Form einer Lösung oder teilweise oder vollständig in Form einer Suspension in der wäßrigen Phase D vorliegt (vorliegen).

**2.** Mittel nach Anspruch 1, dadurch gekennzeichnet, daß das (die) Plättchen der Vesikel bildende amphiphile Lipid (bildenden amphiphilen Lipide) ein ionisches Lipid ist (oder ionische Lipide sind), ausgewählt unter natürlichen oder synthetischen Phosphorlipiden.

**3.** Mittel nach Anspruch 1, dadurch gekennzeichnet, daß das (die) Plättchen der Vesikel bildende amphiphile Lipid (bildenden amphiphilen Lipide) ein nicht-ionisches Lipid ist (oder nicht-ionische Lipide sind), ausgewählt unter den Verbindungen der Formel (I)

$$RO-[C_3H_5(OH)O]_{\bar{n}}-OH \qquad (I)$$

- worin - $C_3H_5(OH)O$ die folgenden Strukturen einzeln oder in Kombination repräsentiert: $-CH_2CHOH\ CH_2O-$ ;

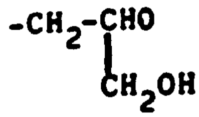 ; 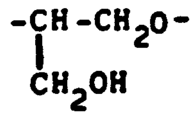

- worin $\bar{n}$ einen statistischen Mittelwert zwischen 2 und 6 bedeutet;
- worin R bedeutet:
  a) eine aliphatische Kette $R_1$ oder einen Rest $R_2CO$, wobei $R_1$ einen aliphatischen, linearen oder verzweigten $C_{12}$-$C_{18}$-Rest bedeutet und $R_2$ einen aliphatischen, linearen oder verzweigten $C_{11}$-$C_{17}$-Rest bedeutet;
  b)

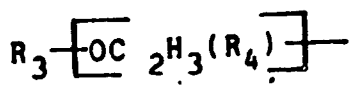

worin $-OC_2H_3(R_4)-$ die folgenden Strukturen einzeln oder in Kombination repräsentiert:

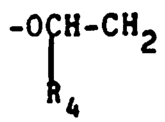 und 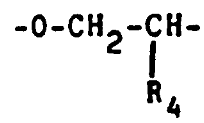

wobei $R_3$ den Rest $R_1$ oder $R_2CO$ bedeutet, $R_4$ einen Rest $R_1$ bedeutet und $R_1$ und $R_2$ die oben angegebenen Bedeutungen besitzen,

und oxyäthylenierten Phytosterolen.

**4.** Mittel nach einem der Ansprüche 1 bis 3, dadurch gekennzeichnet, daß das amphiphile Lipid (die amphiphilen Lipide) der Plättchen mit wenigstens einem Additiv assoziiert ist (sind), das die Permeabilität oder die Oberflächenladung der Vesikel modifiziert.

**5.** Mittel nach einem der Ansprüche 1 bis 4, dadurch gekennzeichnet, daß das Gesamtgewicht des (der) die Vesikel bildenden Lipides (Lipide), gegebenenfalls zusammen mit dem (oder den) damit assoziierten Additiv(en), zwischen 0,2 und 20% des Gesamtgewichtes des Mittels ausmacht.

**6.** Mittel nach einem der Ansprüche 1 bis 5, dadurch gekennzeichnet, daß es wenigstens ein Pyrimidinderivat der Formel:

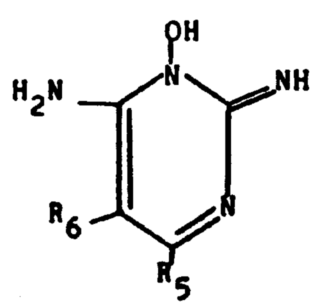

(II)

worin
- $R_5$ eine Gruppe

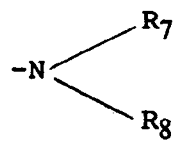

bedeutet, in der $R_7$ und $R_8$ ausgewählt sind unter Wasserstoff, einer $C_1$-$C_4$-Alkylgruppe, $C_2$-$C_5$-Alkenylgruppe, ($C_1$-$C_4$-Alkyl)arylgruppe und $C_4$-$C_7$-Cycloalkylgruppe oder mit dem Stickstoffatom, an das sie gebunden sind, einen Heterozyklus bilden, der ausgewählt ist unter Aziridinyl-, Azetidinyl-, Pyrrolidinyl-, Piperidinyl-, Hexahydroazepinyl-, Heptamethylenimin-, Octamethylenimin-, Morpholin- oder 4- ($C_1$-$C_4$-Alkyl)-piperazinyl-Gruppen, wobei die heterozyklischen Gruppen an den Kohlenstoffatomen durch 1 bis 3 $C_1$-$C_4$-Alkyl-, Hydroxy-oder $C_1$-$C_4$-Alkoxygruppen substituiert sein können;

- $R_6$ ein Wasserstoffatom, eine $C_1$-$C_4$-Alkylgruppe, $C_2$-$C_5$-Alkenylgruppe, $C_4$-$C_7$-Cycloalkylgruppe, Arylgruppe, ($C_1$-$C_4$-Alkyl)arylgruppe, Aryl($C_1$-$C_4$-alkyl)gruppe, ($C_1$-$C_4$-Alkyl)aryl ($C_1$-$C_4$-alkyl)gruppe, Alkoxy($C_1$-$C_4$-alkyl)gruppe, ($C_1$-$C_4$-Alkoxy)aryl($C_1$-$C_4$-alkyl)gruppe oder Halogenaryl($C_1$-$C_4$-alkyl)gruppe bedeutet und/oder wenigstens ein physiologisch akzeptables Säureadditionssalz enthält.

**7.** Mittel nach Anspruch 6, dadurch gekennzeichnet, daß es 6-Amino-1,2-dihydro-1-hydroxy-2-imino-4-piperidinopyrimidin und/oder wenigstens ein physiologisch akzeptables Säureadditionssalz davon enthält.

**8.** Mittel nach einem der Ansprüche 1 bis 7, dadurch gekennzeichnet, daß es wenigstens ein Pyrimidinderivat enthält, das wenigstens teilweise in Form einer Suspension in der wäßrigen Phase D vorliegt, wobei die Partikel eine Teilchengröße von weniger als 80 µm aufweisen.

**9.** Mittel nach einem der Ansprüche 1 bis 8, dadurch gekennzeichnet, daß das Pyrimidinderivat (die Pyrimidinderivate) in dem Mittel in einem Anteil zwischen 0,05 und 6 Gew.-%, bezogen auf das Gesamtgewicht des Mittels, vorhanden ist (sind).

**10.** Mittel nach einem der Ansprüche 1 bis 9, dadurch gekennzeichnet, daß es sich bei dem Retinoidderivat um ein Derivat der Formel (III):

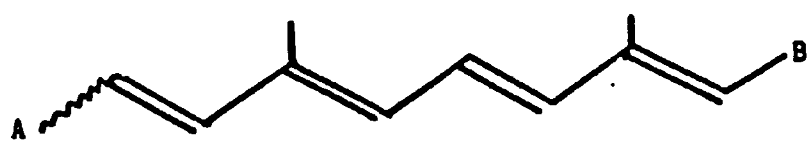

(III)

worin:

a) A ausgewählt ist unter den Gruppen der Formeln:

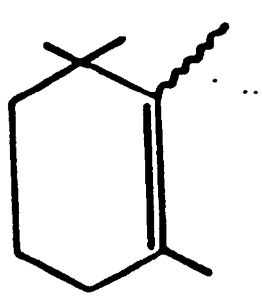

(IVa)

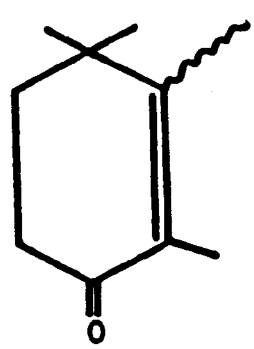

(V)

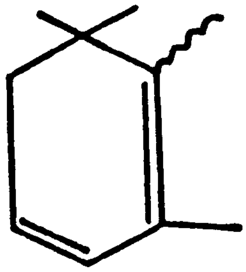

(IVb)

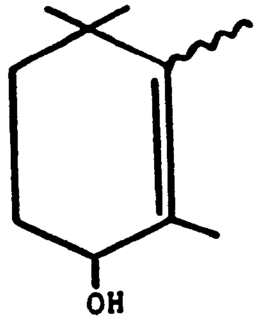

(VI)

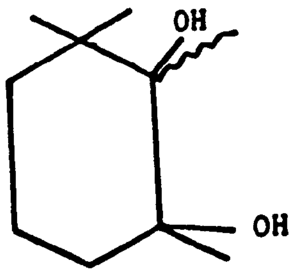

(VII)

wobei B dann ausgewählt ist unter:

aa) wenn A eine Gruppe der Formel IVa ist:

- den folgenden Gruppen:

. -CHO;

. $-CH_2OR_9$, worin $R_9$ für Wasserstoff oder einen $C_1$-$C_4$-Alkylrest steht;

.

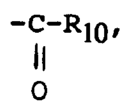

worin $R_{10}$ einen linearen oder verzweigten $C_1$-$C_{16}$-Alkylrest bedeutet;

. $-CH_2SR_{11}$, worin $R_{11}$ für Wasserstoff oder einen Methylrest steht;

.

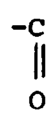

X, worin X bedeutet:

(i) -OH;

(ii) $-OR_{12}$, worin $R_{12}$ einen $C_1$-$C_{15}$-Alkylrest, Aryl($C_1$-$C_4$-alkyl)rest, der an der Arylgruppe gegebenenfalls substituiert sein kann, Arylcarboxy($C_1$-$C_4$-alkyl)rest, der an der Arylgruppe gegebenenfalls substituiert sein kann, Hydroxy($C_1$-$C_4$-alkyl) rest, Amido($C_1$-$C_4$-alkyl)rest bedeutet;

(iii) $NR_{13}R_{14}$, worin $R_{13}$ oder $R_{14}$, die gleich oder verschieden sind, für Wasserstoff, einen $C_1$-$C_6$-Alkylrest, Hydroxy-$C_1$-$C_4$-Alkylrest, substituierten oder unsubstituierten Arylrest, substituierten oder unsubstituierten Heterozyklus bedeuten oder worin $R_{13}$ und $R_{14}$ zusammen mit dem Stickstoffatom, an das sie gebunden sind, einen substituierten oder unsubstituierten Heterozyklus bilden;

(iv) die Gruppe $N_3$;

. $-CH_2NHR_{15}$, worin $R_{15}$ einen substituierten oder unsubstituierten Benzoylrest bedeutet;
ab) wenn A eine Gruppe der Formel IVb, V, VI oder VII bedeutet:
-COOH und den damit gebildeten Salzen oder
Estern;
b) oder A ausgewählt ist unter substituierten oder unsubstituierten Arylgruppen, substituierten oder unsubstituierten Heterozyklen, arylheterozyklischen Gruppen, die am Heterozyklus gegebenenfalls substituiert sein können, oder arylhomozyklischen Gruppen, die am aromatischen Kern gegebenenfalls substituiert sein können,

wobei B dann ausgewählt ist unter: -COOH, $-COOR_{16}$, worin $R_{16}$ einen $C_1$-$C_4$-Alklyrest oder einen durch einen $C_1$-$C_4$-Alkylrest substiuierten Amidrest bedeutet,
sowie um die physiologisch akzeptablen Salze und Ester davon handelt.

**11.** Mittel nach Anspruch 10, dadurch gekennzeichnet, daß es sich bei dem Retinoidderivat um ein Derivat der Formel (III) handelt, bei der $C_1$-$C_4$-Alkyl für Methyl, Äthyl, n-Butyl, t-Butyl steht; $C_1$-$C_{16}$-Alkyl für Äthyl, Propyl, Palmityl steht; Aryl für Phenyl oder Benzyl steht, die Substituenten der Arylgruppe $C_1$-$C_4$-Alkyl, $C_1$-$C_{12}$-Alkoxy, Hydroxy, Halogen oder Nitro sind, wobei die Alkyl- oder Alkoxysubstituenten selbst durch eine OH-Gruppe substituiert sein können.

**12.** Mittel nach Anspruch 10, dadurch gekennzeichnet, daß das Retinoidderivat ein Derivat der Formel (III) ist, die eine heterozyklische Gruppe umfaßt, welche ausgewählt ist unter Gruppen, die abgeleitet sind von Phtalimid, Succinimid, und Heterozyklen mit 4 bis 6 Ringgliedern, welche ein oder mehrere Sauerstoffatome und/oder ein oder mehrere Stickstoffatome umfassen.

**13.** Mittel nach Anspruch 10, dadurch gekennzeichnet, daß das Retinoidderivat ausgewählt ist unter Retinal; Retinol; Retinylacetat, -propionat und -palmitat; Retinoesäure in den Formen all-trans, 13-cis, 9-cis, 11-cis, 9,13-di-cis, 11,13-di-cis; den entsprechenden Zinkretinoaten, Motretinid; Etretinat; quaternären Ammoniumretinoaten der Formel:

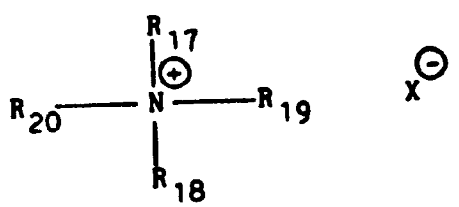

(VIII)

worin:
$X^\ominus$ einen all-trans- oder 13-cis-Retinoatrest bedeutet und
i) $R_{17}$, $R_{18}$, $R_{19}$, die gleich oder verschieden sind, einen linearen $C_1$-$C_4$-Alkylrest, der durch ein oder mehrere Hydroxyreste substituiert sein kann, bedeuten, $R_{20}$ einen linearen $C_{12}$-$C_{18}$-Alkyl-oder -Alkenylrest bedeutet;
ii) oder $R_{19}$ eine Gruppe

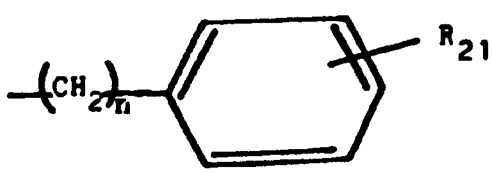

bedeutet, worin:
n für 0 oder 1 steht;
$R_{21}$ ein Wasserstoff- oder Halogenatom, einen Hydroxyrest, $C_1$-$C_{18}$-Alkyl- oder Hydroxyalkylrest oder $C_2$-$C_{18}$-Acylrest bedeutet;

$R_{17}$, $R_{18}$ und $R_{19}$ die unter i) angegebenen Bedeutungen besitzen;
iii) oder $R_{17}$ und $R_{18}$ einen aliphatischen Heterozyklus bilden, der wenigstens ein Sauerstoff-, Stickstoff- oder Schwefelatom umfaßt, $R_{19}$ und $R_{20}$ die unter i) und ii) angebenen Bedeutungen besitzen.

**14.** Mittel nach Anspruch 1, dadurch gekennzeichnet, daß es als Pyrimidinderivat Minoxidil und als Retinoidderivat all-trans-Retinoesäure enthält.

**15.** Mittel nach einem der Ansprüche 1 bis 14, dadurch gekennzeichnet, daß das Retinoidderivat der Formel (III), worin B für -COOH steht, in einem Anteil zwischen 0,005 und 0,030 Gew.-%, bezogen auf das Gesamtgewicht des Mittels, vorhanden ist.

**16.** Mittel nach einem der Ansprüche 1 bis 15, dadurch gekennzeichnet, daß die wäßrige Phase D geliert ist.

**17.** Mittel nach Anspruch 16, dadurch gekennzeichnet, daß das gelbildende Mittel ausgewählt ist unter Heterobiopolysacchariden, Cellulosederivaten und vernetzten oder unvernetzten Acrylpolymeren.

**18.** Mittel nach einem der Ansprüche 16 oder 17, dadurch gekennzeichnet, daß das gelbildende Mittel in einem Anteil zwischen 0,1 und 5 Gew.-%, bezogen auf das Gesamtgewicht des Mittels, vorhanden ist.

**19.** Mittel nach einem der Ansprüche 1 bis 18, dadurch gekennzeichnet, daß die wäßrige Phase D wenigstens ein wasserlösliches, physiologisch akzeptables Additiv enthält.

**20.** Mittel nach einem der Ansprüche 1 bis 19, dadurch gekennzeichnet, daß sein pH zwischen 4 und 9 liegt.

**21.** Verwendung eines Mittels nach einem der Ansprüche 1 bis 20 zur Herstellung eines Medikamentes, das dazu bestimmt ist, das Wachstum der Haare zu induzieren und stimulieren und Haarausfall zu verringern.

**22.** Verfahren zur kosmetischen Behandlung der Haare und der Kopfhaut, dadurch gekennzeichnet, daß man auf diese 1 bis 5 g des Mittels nach einem der Ansprüche 1 bis 20 ein bis zweimal pro Tag während 1 bis 7 Tagen pro Woche und während eines Behandlungszeitraums zwischen 1 und 6 Monaten durch Einreiben aufträgt.

**Patentansprüche für folgende Vertragsstaaten : ES, GR**

**1.** Kosmetisches Mittel zur topischen Anwendung, enthaltend in Kombination wenigstens ein Retinoidderivat und wenigstens ein Pyrimidinderivat, dadurch gekennzeichnet, daß es aus einer Dispersion von Vesikeln aus wenigstens einem ionischen und/oder nichtionischen amphiphilen Lipid, welche durch ein oder mehrere Lipidplättchen begrenzt sind, in einer wäßrigen physiologisch akzeptablen Dispersionsphase D gebildet ist, wobei sich das Retinoidderivat (die Retinoidderivate) in den Lipidplättchen der Vesikel befindet (befinden), während das Pyrimidinderivat (die Pyrimidinderivate) in Form einer Lösung oder teilweise oder vollständig in Form einer Suspension in der wäßrigen Phase D vorliegt (vorliegen).

**2.** Mittel nach Anspruch 1, dadurch gekennzeichnet, daß das (die) Plättchen der Vesikel bildende amphiphile Lipid (bildenden amphiphilen Lipide) ein ionisches Lipid ist (oder ionische Lipide sind), ausgewählt unter natürlichen oder synthetischen Phosphorlipiden.

**3.** Mittel nach Anspruch 1, dadurch gekennzeichnet, daß das (die) Plättchen der Vesikel bildende amphiphile Lipid (bildenden amphiphilen Lipide) ein nicht-ionisches Lipid ist (oder nicht-ionische Lipide sind), ausgewählt unter den Verbindungen der Formel (I)

$$RO-[C_3H_5(OH)O]_{\bar{n}}-OH \quad (I)$$

- worin - $C_3H_5(OH)O$ die folgenden Strukturen einzeln oder in Kombination repräsentiert:
  $-CH_2CHOH\ CH_2O-$ ;

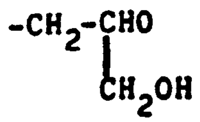 ; 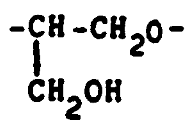

- worin $\bar{n}$ einen statistischen Mittelwert zwischen 2 und 6 bedeutet;
- worin R bedeutet:

  a) eine aliphatische Kette $R_1$ oder einen Rest $R_2CO$, wobei $R_1$ einen aliphatischen, linearen oder verzweigten $C_{12}$-$C_{18}$-Rest bedeutet und $R_2$ einen aliphatischen, linearen oder verzweigten $C_{11}$-$C_{17}$-Rest bedeutet;

  b)

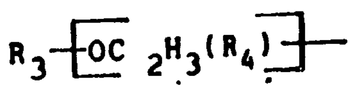

worin $-OC_2H_3(R_4)-$ die folgenden Strukturen einzeln oder in Kombination repräsentiert:

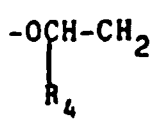 und 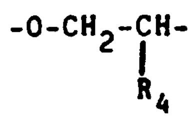

wobei $R_3$ den Rest $R_1$ oder $R_3CO$ bedeutet, $R_4$ einen Rest $R_1$ bedeutet und $R_1$ und $R_2$ die oben angegebenen Bedeutungen besitzen,

und oxyäthylenierten Phytosterolen.

4. Mittel nach einem der Ansprüche 1 bis 3, dadurch gekennzeichnet, daß das amphiphile Lipid (die amphiphilen Lipide) der Plättchen mit wenigstens einem Additiv assoziiert ist (sind), das die Permeabilität oder die Oberflächenladung der Vesikel modifiziert.

5. Mittel nach einem der Ansprüche 1 bis 4, dadurch gekennzeichnet, daß das Gesamtgewicht des (der) die Vesikel bildenden Lipides (Lipide), gegebenenfalls zusammen mit dem (oder den) damit assoziierten Additiv(en), zwischen 0,2 und 20% des Gesamtgewichtes des Mittels ausmacht.

6. Mittel nach einem der Ansprüche 1 bis 5, dadurch gekennzeichnet, daß es wenigstens ein Pyrimidinderivat der Formel:

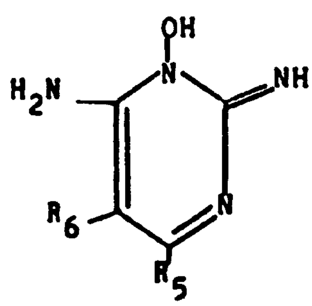

(II)

worin

- $R_5$ eine Gruppe

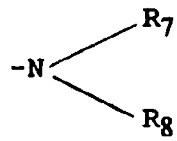

bedeutet, in der $R_7$ und $R_8$ ausgewählt sind unter Wasserstoff, einer $C_1$-$C_4$-Alkylgruppe, $C_2$-$C_5$-Alkenylgruppe, ($C_1$-$C_4$-Alkyl) arylgruppe und $C_4$-$C_7$-Cycloalkylgruppe oder mit dem Stickstoffatom, an das sie gebunden sind, einen Heterozyklus bilden, der ausgewählt ist unter Aziridinyl-, Azetidinyl-, Pyrrolidinyl-, Piperidinyl-, Hexahydroazepinyl-, Heptamethylenimin-, Octamethylenimin-, Morpholin- oder 4-($C_1$-$C_4$-Alkyl)-piperazinyl-Gruppen, wobei die heterozyklischen Gruppen an den Kohlenstoffatomen durch 1 bis 3 $C_1$-$C_4$-Alkyl-, Hydroxy-oder $C_1$-$C_4$-Alkoxygruppen substituiert sein können;

- $R_6$ ein Wasserstoffatom, eine $C_1$-$C_4$-Alkylgruppe, $C_2$-$C_5$-Alkenylgruppe, $C_4$-$C_7$-Cycloalkylgruppe, Arylgruppe, ($C_1$-$C_4$-Alkyl)arylgruppe, Aryl($C_1$-$C_4$-alkyl)gruppe, ($C_1$-$C_4$-Alkyl)aryl($C_1$-$C_4$-alkyl)gruppe, Alkoxy($C_1$-$C_4$-alkyl) gruppe, ($C_1$-$C_4$-Alkoxy)aryl($C_1$-$C_4$-alkyl)gruppe oder Halogenaryl($C_1$-$C_4$-alkyl)gruppe bedeutet und/oder wenigstens ein physiologisch akzeptables Säureadditionssalz enthält.

7. Mittel nach Anspruch 6, dadurch gekennzeichnet, daß es 6-Amino-1,2-dihydro-1-hydroxy-2-imino-4-piperidino-pyrimidin und/oder wenigstens ein physiologisch akzeptables Säureadditionssalz davon enthält.

8. Mittel nach einem der Ansprüche 1 bis 7, dadurch gekennzeichnet, daß es wenigstens ein Pyrimidinderivat enthält, das wenigstens teilweise in Form einer Suspension in der wäßrigen Phase D vorliegt, wobei die Partikel eine Teilchengröße von weniger als 80 µm aufweisen.

9. Mittel nach einem der Ansprüche 1 bis 8, dadurch gekennzeichnet, daß das Pyrimidinderivat (die Pyrimidinderivate) in dem Mittel in einem Anteil zwischen 0,05 und 6 Gew.-%, bezogen auf das Gesamtgewicht des Mittels, vorhanden ist (sind).

10. Mittel nach einem der Ansprüche 1 bis 9, dadurch gekennzeichnet, daß es sich bei dem Retinoidderivat um ein Derivat der Formel (III):

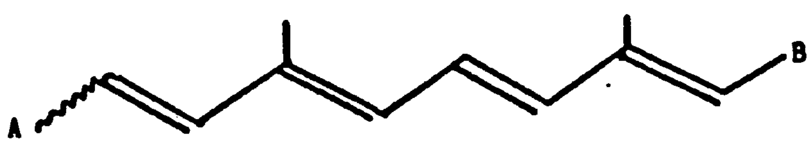

(III)

worin:

a) A ausgewählt ist unter den Gruppen der Formeln:

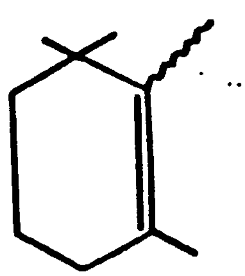

(IVa)

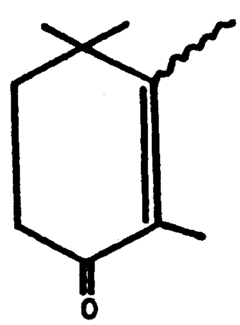

(V)

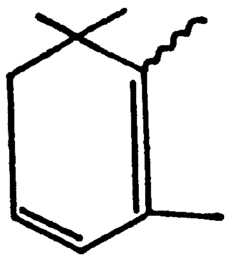

(IVb)

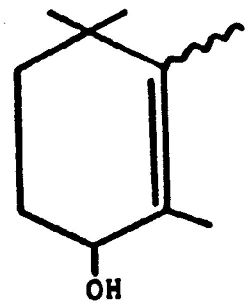

(VI)

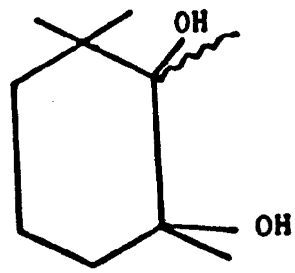

(VII)

wobei B dann ausgewählt ist unter:

aa) wenn A eine Gruppe der Formel IVa ist:

- den folgenden Gruppen:

. -CHO;

. $-CH_2OR_9$, worin $R_9$ für Wasserstoff oder einen $C_1$-$C_4$-Alkylrest steht;

.

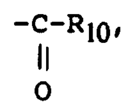

worin $R_{10}$ einen linearen oder verzweigten $C_1$-$C_{16}$-Alkylrest bedeutet;

. $-CH_2SR_{11}$, worin $R_{11}$ für Wasserstoff oder einen Methylrest steht;

.

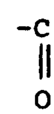

X, worin X bedeutet:

(i) -OH;

(ii) $-OR_{12}$, worin $R_{12}$ einen $C_1$-$C_{15}$-Alkylrest, Aryl($C_1$-$C_4$-alkyl)rest, der an der Arylgruppe gegebenenfalls substituiert sein kann, Arylcarboxy($C_1$-$C_4$-alkyl)rest, der an der Arylgruppe gegebenenfalls substituiert sein kann, Hydroxy($C_1$-$C_4$-alkyl)rest, Amido($C_1$-$C_4$-alkyl)rest bedeutet;

(iii) $NR_{13}R_{14}$, worin $R_{13}$ oder $R_{14}$, die gleich oder verschieden sind, für Wasserstoff, einen $C_1$-$C_6$-Alkylrest, Hydroxy-$C_1$-$C_4$-Alkylrest, substituierten oder unsubstituierten Arylrest, substituierten oder unsubstituierten Heterozyklus bedeuten oder worin $R_{13}$ und $R_{14}$ zusammen mit dem stickstoffatom, an das sie gebunden sind, einen substituierten oder unsubstituierten Heterozyklus bilden;

(iv) die Gruppe $N_3$;

. $CH_2NHR_{15}$ , worin $R_{15}$ einen substituierten oder unsubstituierten Benzoylrest bedeutet;
ab) wenn A eine Gruppe der Formel IVb, V, VI oder VII bedeutet:
-COOH und den damit gebildeten Salzen oder
Estern;
b) oder A ausgewählt ist unter substituierten oder unsubstituierten Arylgruppen, substituierten oder unsubstituierten Heterozyklen, arylheterozyklischen Gruppen, die am Heterozyklus gegebenenfalls substituiert sein können, oder arylhomozyklischen Gruppen, die am aromatischen Kern gegebenenfalls substituiert sein können,

wobei B dann ausgewählt ist unter: -COOH, $-COOR_{16}$, worin $R_{16}$ einen $C_1$-$C_4$-Alklyrest oder einen durch einen $C_1$-$C_4$-Alkylrest substiuierten Amidrest bedeutet, sowie um die physiologisch akzeptablen Salze und Ester davon handelt.

**11.** Mittel nach Anspruch 10, dadurch gekennzeichnet, daß es sich bei dem Retinoidderivat um ein Derivat der Formel (III) handelt, bei der $C_1$-$C_4$-Alkyl für Methyl, Äthyl, n-Butyl, t-Butyl steht; $C_1$-$C_{16}$-Alkyl für Äthyl, Propyl, Palmityl steht; Aryl für Phenyl oder Benzyl steht, die Substituenten der Arylgruppe $C_1$-$C_4$-Alkyl, $C_1$-$C_{12}$-Alkoxy, Hydroxy, Halogen oder Nitro sind, wobei die Alkyl- oder Alkoxysubstituenten selbst durch eine OH-Gruppe substituiert sein können.

**12.** Mittel nach Anspruch 10, dadurch gekennzeichnet, daß das Retinoidderivat ein Derivat der Formel (III) ist, die eine heterozyklische Gruppe umfaßt, welche ausgewählt ist unter Gruppen, die abgeleitet sind von Phtalimid, succinimid, und Heterozyklen mit 4 bis 6 Ringgliedern, welche ein oder mehrere Sauerstoffatome und/oder ein oder mehrere Stickstoffatome umfassen.

**13.** Mittel nach Anspruch 10, dadurch gekennzeichnet, daß das Retinoidderivat ausgewählt ist unter Retinal; Retinol; Retinylacetat, -propionat und -palmitat; Retinoesäure in den Formen all-trans, 13-cis, 9-cis, 11-cis, 9,13-di-cis, 11,13-di-cis; den entsprechenden Zinkretinoaten, Motretinid; Etretinat; quaternären Ammoniumretinoaten der Formel:

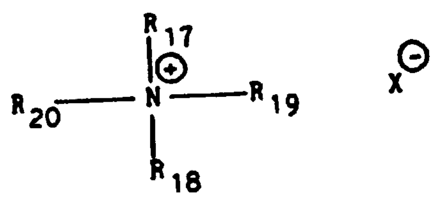

(VIII)

worin:
X⊖ einen all-trans- oder 13-cis-Retinoatrest bedeutet und

i) $R_{17}$, $R_{18}$, $R_{19}$, die gleich oder verschieden sind, einen linearen $C_1$-$C_4$-Alkylrest, der durch ein oder mehrere Hydroxyreste substituiert sein kann, bedeuten, $R_{20}$ einen linearen $C_{12}$-$C_{18}$-Alkyl-oder-Alkenylrest bedeutet;
ii) oder $R_{19}$ eine Gruppe

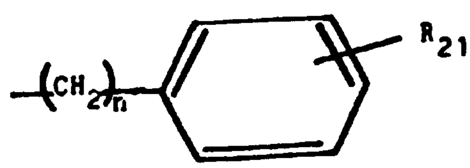

bedeutet, worin:
n für 0 oder 1 steht;
$R_{21}$ ein Wasserstoff- oder Halogenatom, einen Hydroxyrest, $C_1$-$C_{18}$-Alkyl- oder Hydroxyalkylrest oder $C_2$-$C_{18}$-Acylrest bedeutet;
$R_{17}$, $R_{18}$ und $R_{19}$ die unter i) angegebenen Bedeutungen besitzen;

iii) oder $R_{17}$ und $R_{18}$ einen aliphatischen Heterozyklus bilden, der wenigstens ein Sauerstoff-, Stickstoff- oder Schwefelatom umfaßt, $R_{19}$ und $R_{20}$ die unter i) und ii) angebenen Bedeutungen besitzen.

**14.** Mittel nach Anspruch 1, dadurch gekennzeichnet, daß es als Pyrimidinderivat Minoxidil und als Retinoidderivat all-trans-Retinoesäure enthält.

**15.** Mittel nach einem der Ansprüche 1 bis 14, dadurch gekennzeichnet, daß das Retinoidderivat der Formel (III), worin B für -COOH steht, in einem Anteil zwischen 0,005 und 0,030 Gew.-%, bezogen auf das Gesamtgewicht des Mittels, vorhanden ist.

**16.** Mittel nach einem der Ansprüche 1 bis 15, dadurch gekennzeichnet, daß die wäßrige Phase D geliert ist.

**17.** Mittel nach Anspruch 16, dadurch gekennzeichnet, daß das gelbildende Mittel ausgewählt ist unter Heterobiopolysacchariden, Cellulosederivaten und vernetzten oder unvernetzten Acrylpolymeren.

**18.** Mittel nach einem der Ansprüche 16 oder 17, dadurch gekennzeichnet, daß das gelbildende Mittel in einem Anteil zwischen 0,1 und 5 Gew.-%, bezogen auf das Gesamtgewicht des Mittels, vorhanden ist.

**19.** Mittel nach einem der Ansprüche 1 bis 18, dadurch gekennzeichnet, daß die wäßrige Phase D wenigstens ein wasserlösliches, physiologisch akzeptables Additiv enthält.

**20.** Mittel nach einem der Ansprüche 1 bis 19, dadurch gekennzeichnet, daß sein pH zwischen 4 und 9 liegt.

**21.** Verfahren zur kosmetischen Behandlung der Haare und der Kopfhaut, dadurch gekennzeichnet, daß man auf diese 1 bis 5 g des Mittels nach einem der Ansprüche 1 bis 20 ein bis zweimal pro Tag während 1 bis 7 Tagen pro Woche und während eines Behandlungszeitraums zwischen 1 und 6 Monaten durch Einreiben aufträgt.